# EUROPÄISCHE PATENTSCHRIFT

(11) **EP 3 876 819 B1**
(45) Veröffentlichungstag und Bekanntmachung des Hinweises auf die Patenterteilung: **20.12.2023**
(21) Anmeldenummer: 19812673.2
(22) Anmeldetag: 07.11.2019
(51) Int. Cl.: A61B 1/015, A61B 1/018, A61B 1/005, A61B 1/00, A61M 25/01, A61M 1/00

(54) **ENDOSKOPISCHES INSTRUMENT**
ENDOSCOPIC INSTRUMENT
INSTRUMENT ENDOSCOPIQUE

(30) Priorität: 07.11.2018 DE 102018218954
(43) Veröffentlichungstag der Anmeldung: 15.09.2021
(73) Patentinhaber: Richard Wolf GmbH, 75438 Knittlingen (DE)
(72) Erfinder: HEIMBERGER, Rudolf, 75038 Oberderdingen (DE)
(74) Vertreter: Patentanwälte Vollmann Hemmer Lindfeld Partnerschaft mbB
(86) Internationale Anmeldenummer: PCT/DE2019/200127
(87) Internationale Veröffentlichungsnummer: WO 2020/094190

(56) Entgegenhaltungen:
- US-A1- 2016 096 004
- US-A1- 2016 100 745
- US-A1- 2017 238 793

## Beschreibung

Die Offenbarung betrifft ein endoskopisches Instrument zum Einführen in einen Körper eines Patienten, vorzugsweise als Einwegartikel zum Entsorgen nach einmaliger Verwendung und vorzugsweise zur minimalinvasiven Diagnostik von Nieren- und Harnleitern sowie zur Nieren- oder Harnsteinentfernung.

Ausgangspunkt der Erfindung sind Ureterorenoskope wie sie etwa aus der EP 2 986 237 B1 bekannt sind. Ein Schaft des Ureterorenoskops wird über den Harnleiter in den Körper eines Patienten eingeführt, um mit einem distalen Greifmittel wie etwa einem Fangkorb oder einer Dormia-Schlinge einen Nieren- oder Harnstein einzufangen und zu entfernen. Solche Ureterorenoskope können allerdings nicht nur therapeutisch, sondern auch lediglich diagnostisch Verwendung finden, da die behandelnde Person damit Nieren- oder Harnleiter direkt einsehen und diagnostizieren kann. Üblicherweise wird die Sichtbarkeit durch Spülflüssigkeit verbessert, die durch eine durch den Schaft geführte Spülleitung am distalen Ende des Schafts austritt und die Sicht behinderndes Gewebe fortschwemmt.

Es gibt die ständige Bestrebung, den Schaftdurchmesser solcher Instrumente zu verringern, um den medizinischen Eingriff so minimalinvasiv wie möglich zu gestalten, oder den zur Verfügung stehenden Querschnitt besser auszunutzen, um bei gleichem Querschnitt mehr oder verbesserte Funktionalitäten bieten zu können.

Das hierin offenbarte endoskopische Instrument kann mit einem geringeren Schaftdurchmesser ausgestaltet werden als bekannte endoskopische Instrumente dieser Art oder nutzt den zur Verfügung stehenden Querschnitt besser aus, um bei gleichem Querschnitt mehr oder verbesserte Funktionalitäten anbieten zu können.

Gemäß einem Aspekt der vorliegenden Offenbarung wird ein endoskopisches Instrument zum Einführen in einen Körper eines Patienten bereitgestellt, wobei das Instrument einen rohrförmigen Schaft und mindestens zwei durch den Schaft verlaufende elektrische, mechanische und/oder optische Leitungen aufweist. Dabei weist der Schaft in einem krümmbaren Schaftabschnitt eine Mehrzahl von Schlitzen auf. Die Schlitze erstrecken sich dabei in Umfangsrichtung nur über einen Teil des Schaftumfangs. Damit wird die Flexibilität des Schafts lokal erhöht, also der Verbiegeradius lokal reduziert, um ein gelenkloses Abwinkeln eines distalen Schaftendes um bis zu 300° zu erzielen. Die Schlitze sind außerdem axial zueinander derart angeordnet, dass sie abwechselnd auf einer ersten lateralen Seite des Schafts und einer diametral der ersten gegenüberliegenden zweiten lateralen Seite des Schafts liegen. Damit kann das distale Schaftende in zwei entgegengesetzte Richtungen gelenklos abgewinkelt werden und bei entsprechend unabhängig voneinander betätigbaren Seilzügen sogar S-förmig zwei entgegengesetzte Krümmungen vollführen. Für die Krümmung zu einer ersten Seite hin werden die Schlitze auf der ersten Seite zusammengedrückt und die Schlitze auf einer der ersten Seite gegenüberliegenden zweiten Seite auseinandergezogen. Entsprechend werden für die Krümmung zur zweiten Seite hin die Schlitze auf der zweiten Seite zusammengedrückt und die die Schlitze auf der ersten Seite auseinandergezogen. Die Seilzüge verlaufen dann vorzugsweise an der ersten bzw. der zweiten Seite im Schaft entlang und greifen am distalen Schaftende an, um das distale Schaftende durch Zugkraft zur entsprechenden Seite hin gelenklos abzuwinkeln.

Optional kann im Schaft ein die mindestens zwei durch den Schaft verlaufenden Leitungen jeweils einzeln unmittelbar umgebender Fluidkanal ausgebildet sein.

"Einzeln unmittelbar umgeben" soll hier bedeuten, dass die Leitungen keine gemeinsame Ummantelung innerhalb des Fluidkanals aufweisen, sondern einzeln vom Fluid im Fluidkanal unmittelbar umspült sind. Die Leitungen können jede für sich ummantelt und beispielsweise isoliert sein. "Unmittelbar" soll hier also nicht dahingehend missverstanden werden, dass die Leitungen für sich selbst keine Ummantelung haben dürfen. Die Leitungen können optischer Natur sein, beispielsweise als Lichtleiter zum Aus- und/oder Einkoppeln von Licht am distalen Ende. Insbesondere können die Leitungen einen oder mehrere Laserlichtleiter aufweisen, um beispielsweise einen Nieren- oder Harnstein mit Laserlicht beschießen und damit vaporisieren zu können. Alternativ oder zusätzlich können eine oder mehrere der Leitungen elektrische Signale und/oder elektrische Leistung übertragen und mit einer distalen LED und/oder einem distalen Bildsensor verbunden sein. Alternativ oder zusätzlich können die Leitungen ein oder mehr Arbeitskanäle aufweisen, durch die Schaftwerkzeuge und/oder ein Laserlichtleiter bis zur distalen Schaftspitze durchgeschoben werden können. Alternativ oder zusätzlich können die Leitungen mechanische Steuersignale zum gelenklosen Abwinkeln und/oder Steuern des distalen Schaftendes übertragen, beispielsweise als seitlich im Schaft geführte Seilzüge. Sämtliche Leitungen sind jeweils einzeln unmittelbar vom Fluidkanal umgeben. "Umgeben" soll hier bedeuten, dass die Leitungen zumindest zum Großteil im Querschnitt umspült sind. Sie müssen nicht im Querschnitt vollständig um 360° umspült sein, sondern können lateral am Schaft oder an einer oder mehreren der anderen Leitungen anliegen.

Zum einen kann bei dem hierin offenbarten endoskopischen Instrument eine durch den Schaft geführte Fluidleitung eingespart werden. Außerdem wird der gesamte Querschnitt des Innenvolumens des Schafts, den die Leitungen nicht belegen, als Fluidkanal nutzbar gemacht, sodass der zur Verfügung stehende Querschnitt besser ausgenutzt ist. Damit kann ein Schaftaußendurchmesser von 3mm oder weniger realisiert werden kann.

Optional können die Schlitze einerseits als eine distale Fluidkanalöffnung für den Fluidkanal und andererseits zur lokalen Erhöhung der Flexibilität des Schafts zum gelenklosen Abwinkeln eines distalen Schaftendes dienen. Die Spülflüssigkeit kann dann proximal vom distalen Schaftende lateral aus dem Schafts austreten. Dies hat unter anderem den Vorteil, dass am distalen Schaftende mehr Querschnitt für Funktionen wie etwa einen Bildsensor und/oder mindestens eine Beleuchtungs-LED zur Verfügung stehen.

Optional kann der Fluidkanal als Zu- und/oder Abführkanal dienen. Je nach Bedarf kann wahlweise Spülflüssigkeit zur Verbesserung der Sicht am distalen Schaftende distalwärts zugeführt werden oder Spülflüssigkeit mit störender Gewebesuspension proximalwärts abgeführt werden. Eine Rückführung von Fluid proximalwärts kann durch aktive Ansaugung oder passiv ohne aktive Ansaugung erfolgen, beispielsweise durch Überdruck im Körper des Patienten.

Optional kann der Fluidkanal eine distale Fluidkanalöffnung und eine proximale Fluidkanalöffnung aufweisen, wobei die proximale Fluidkanalöffnung lateral am Schaft angeordnet und mit Fluiddruck oder Fluidunterdruck beaufschlagbar ist. Dazu kann eine Druck- bzw. Saugpumpe an die proximale Fluidkanalöffnung angeschlossen werden oder einfach wie bei einem Tropf ein in einer Höhe über dem Instrument befindliches Fluidreservoir angeschossen sein, sodass ein hydrostatischer Druck an der proximalen Fluidkanalöffnung anliegt. Als Spülflüssigkeit kann beispielsweise Kochsalzlösung dienen.

Optional kann das Instrument eine Dichtungsvorrichtung aufweisen, welches ein proximales Ende des Fluidkanals bildet und Durchführungen für die Leitungen aufweist. Die Dichtungsvorrichtung kann beispielsweise einen Elastomer-Zellschaumblock aufweisen, der in einem proximalen Endbereich des Schafts und/oder in einer mit einem proximalen Endbereich des Schafts verbundenen oder verbindbaren Handhabungseinrichtung angeordnet ist. Bevorzugt ist der Zellschaumblock geschlossenzellig ausgeführt. Die Leitungen können durch Durchführungen durch die Dichtungsvorrichtung geführt sein, wobei die Durchführungen jeweils einen ursprünglich geringeren Durchmesser haben als die zugehörige durchgeführte Leitung, allerdings durch die durchgeführte Leitung elastisch aufgeweitet sind. Dadurch kann ein Abdichtungseffekt erzielt werden, sodass kein Fluid durch die Durchführungen proximalwärts axial aus dem Schaft nach außen oder in die Handhabungseinrichtung strömen kann. Das Fluid soll nämlich vorzugsweise nicht axial, sondern lateral durch eine dafür vorgesehene proximale Fluidkanalöffnung strömen, die lateral am Schaft und vorzugsweise distal von der Dichtungsvorrichtung angeordnet ist.

Optional kann das Instrument eine Handhabungseinrichtung aufweisen, die mit einem proximalen Ende des Schafts fest verbunden oder lösbar verbindbar ist. Die Handhabungseinrichtung kann vorzugsweise ergonomisch so gestaltet sein, dass sie von einer behandelnden Person bequem gegriffen werden kann und gut in der Hand liegt, um das Instrument manuell zu steuern. Die Handhabungseinrichtung kann dazu im Wesentlichen Y-förmig mit zwei starren zum Schaft hin distalwärts zusammenlaufenden Griffschenkeln ausgestaltet sein. Die Handhabungseinrichtung kann dann entweder so gegriffen werden, dass beide Griffschenkel wie eine Zange umfasst werden oder der Daumenballen zwischen die Griffschenkel geführt wird. Bei der ersten Haltung kann der Zeigefinger zum Ziehen eines Seilzugs unter den Schaft an einem schusswaffenähnlichen Abzug gelegt werden. Bei der zweiten Haltung kann der Zeigefinger zum Ziehen eines oberen Seilzugs über den Schaft an einem oberen Abzug gelegt und der Mittelfinger zum Ziehen eines unteren Seilzugs unter den Schaft an einem unteren Abzug gelegt. Der obere Seilzug und der untere Seilzug können als Abschnitte eines Seilzugs ausgebildet sein, der über eine Führungsrolle umgelenkt ist, die in der Handhabungseinrichtung um eine quer zur Längsachse des Schafts drehbar gelagert ist. Je nach Drehrichtung der Führungsrolle wird der obere oder der untere Seilzug proximalwärts gezogen während der jeweils andere Seilzug distalwärts nachgibt. Der obere Abzug und der untere Abzug können jeweils miteinander über die Führungsrolle unmittelbar verbunden oder mittelbar gekoppelt sein, sodass sie sich bei Betätigung gegenläufig bewegen. Besonders die zweite Haltung ist ergonomisch besonders vorteilhaft, da die Längsachse des Schafts in Wesentlichen koaxial zur Längsachse des Unterarms verläuft, sodass das Instrument bequem durch Supination und Pronation des Unterarms um seine Längsachse gedreht werden kann. Außerdem können durch Betätigung des oberen oder unteren Abzugs mit dem Zeige- bzw. Mittelfinger einfach ein Abknicken des distalen Schaftendes mittels des oberen bzw. Seilzugs in zwei entgegengesetzte Richtungen bewirkt werden. Bei der ersten Haltung kann die Handhabungseinrichtung um 180° umgegriffen werden, um mit dem Zeigefinger den anderen Abzug zu ziehen, oder das Instrument um 180° um seine Längsachse gedreht werden, um ein Abknicken des distalen Schaftendes mittels des oberen bzw. Seilzugs in zwei entgegengesetzte Richtungen bewirkt werden. Die Leitungen können durch einen oder beide der Griffschenkel der Handhabungseinrichtung geführt sein und an einem proximalen Ende des bzw. der Griffschenkel jeweils einen proximalseitigen Anschluss aufweisen. Vorzugsweise ist durch einen Griffschenkel der Handhabungseinrichtung ein Arbeitskanal geführt und andere Leitungen durch den anderen Griffschenkel der Handhabungseinrichtung.

Optional kann der Schaft zumindest in dem krümmbaren Schaftabschnitt flexibel oder semi-flexibel sein. Insbesondere eine hohe Torsionssteifigkeit bei gewisser Biegeflexibilität ist vorteilhaft zur genauen Steuerung des distalen Schaftendes. Die Biegesteifigkeit kann quantitativ dadurch bemessen werden, wie tief das distale Schaftende bei horizontaler Lage des Instruments allein unter der Gewichtskraft des Schafts nach unten hängt. Es hat sich herausgestellt, dass es besonders vorteilhaft ist, wenn das distale Schaftende 5% bis 60% der Schaftlänge bei horizontaler Lage des Instruments allein unter der Gewichtskraft des Schafts nach unten hängt. In diesem Bereich der Biegesteifigkeit ist der Schaft flexibel genug, um möglichst tief und gleichzeitig minimalinvasiv in die Nieren- und Harnleiter vordringen zu können und biegesteif genug, um das distale Schaftende kontrolliert steuern zu können.

Optional kann das Instrument oder zumindest der Schaft als Wegwerfartikel zum Entsorgen nach einmaliger Verwendung ausgestaltet sein. Dies ist eine besonders vorteilhafte Ausführungsform, da die Reinigung des Instruments für weitere Verwendungen entfällt und die Bauteile und Materialien nur für eine einzige Verwendung ausgelegt werden müssen. Die Leitungen, die einzeln unmittelbar vom Fluidkanal umgeben sind, können sehr filigran ausgestaltet und ohne Rücksicht auf die Ausbildung von schlecht reinigbaren Ecken, Kanten und/oder Toträumen durch den Fluidkanal geführt sein. Durch die filigrane Ausgestaltung der Leitungen und des Schafts selbst kann ein Schaftaußendurchmesser von 2,7 mm oder weniger realisiert werden.

Optional kann die Querschnittsfläche des Fluidkanals der Querschnittsfläche eines vom Schaft gebildeten Schaftinnenraums abzüglich der Summe der Querschnittsfläche aller durch den Schaftinnenraum verlaufenden Leitungen entsprechen. Dies bedeutet, dass es im Schaft keine ungenutzte Querschnittsfläche gibt und somit der Schaftinnenraum optimal ausgenutzt wird.

Optional kann das Instrument mindestens einen durch den Schaft verlaufenden und vom Fluidkanal unmittelbar umgebenen Arbeitskanal aufweisen. Solch ein Arbeitskanal kann wahlweise verschieden verwendet werden. Zum einen kann solch ein Arbeitskanal zum Durchführen eines Schaftwerkzeugs, wie etwa ein Fangkorbeinsatz oder ein Zangeninstrument, geeignet sein. Sofern das Instrument nicht bereits eine optische Leitung als einen durch den Fluidkanal geführten Laserlichtleiter aufweist, so kann ein solcher Laserlichtleiter durch vorzugsweise einen weiteren Arbeitskanal von einer proximalen Arbeitskanalöffnung, beispielsweise an einem proximalen Axialende eines Griffschenkels der Handhabungseinrichtung, eingeführt und bis zu einer distalen Arbeitskanalöffnung am distalen Schaftende durch den Arbeitskanal geschoben werden. Mit dem Laserlichtleiter kann Laserlicht eingekoppelt werden, um beispielsweise einen Nieren- oder Harnstein zu vaporisieren und mittels Spülflüssigkeit aus dem Spülkanal auszuschwemmen. Der Laserlichtleiter kann bei Nichtbenutzung wieder aus dem Arbeitskanal herausgezogen werden, um diesen für mögliche andere Verwendungen freizugeben. Beispielsweise kann ein Fangkorbeinsatz oder eine Dormia-Schlingen-Einsatz durch den Arbeitskanal geschoben werden, um damit am distalen Schaftende einen Nieren- oder Harnstein einzufangen. Die proximale Arbeitskanalöffnung, beispielsweise an einem proximalen Axialende eines Griffschenkels der Handhabungseinrichtung, kann einen Luer-Lock Anschluss aufweisen, um einen Mantel des Fangkorbeinsatzes oder des Dormia-Schlingen-Einsatzes gegenüber dem Instrument zu fixieren und durch Schieben eines durch den Mantel führenden Drahts, der distalseitig den Fangkorb oder die Dormia-Schlinge bildet, den Fangkorb oder die Dormia-Schlinge zu öffnen. Der Fangkorb oder die Dormia-Schlinge schließ sich bei Ziehen des Drahtes, wenn der Mantel im Luer-Lock Anschluss arretiert ist. Bevorzugt ist es jedoch, wenn ein erster, vorzugsweise größerer, Arbeitskanal für ein Schaftwerkzeug und ein zweiter, vorzugsweise kleinerer, Arbeitskanal für einen Laserlichtleiter bereitgestellt wird. Dadurch können Schaftwerkzeug und Laserlichtleiter parallel genutzt werden.

Optional kann ein Arbeitskanal als Zu- und/oder Abführkanal mit gegenüber dem Fluidkanal gegenläufiger Strömungsrichtung dienen. Dies ist insbesondere dann von Vorteil, wenn bei längerer Behandlungszeit und größeren Mengen von Spülflüssigkeit diese aus dem Körper wieder abgeführt werden muss, ohne die Behandlung zu unterbrechen. Eine Rückführung von Spülflüssigkeit an der Außenseite des Schafts oder durch einen zusätzlich eingeführten Hilfsschaft führt zu einer zusätzlichen Gewebeaufdehnung und ist im Hinblick auf einen minimalinvasiven Eingriff nachteilhaft. Außerdem kann sich Überdruck aufbauen, der dem Spülmittelfluss entgegenwirkt. Der Arbeitskanal kann vorzugsweise als Zuführleitung von Spülflüssigkeit verwendet werden, die über den Fluidkanal als Abführleitung abgeführt wird. Alternativ kann der Spülmittelfluss umgekehrt verlaufen, sodass der Arbeitskanal als Abführkanal und der Fluidkanal als Zuführkanal dient. Die Verwendung des Arbeitskanals als Zu- und/oder Abführkanal kann ein kontinuierliches Spülen erleichtern.

Optional kann der mindestens eine Arbeitskanal axial durch ein abgedichtetes proximales Ende des Fluidkanals verlaufen. Der Arbeitskanal kann also analog zu den anderen Leitungen durch eine zugehörige axiale Durchführung in der Dichtungsvorrichtung geführt sein, die beispielsweise in Form eines Elastomer-Zellschaumblocks ausgebildet ist.

Optional kann die proximale Arbeitskanalöffnung proximal vom proximalen Ende des Fluidkanals angeordnet sein, vorzugsweise an einem proximalen Axialende eines Griffschenkels der Handhabungseinrichtung. Damit ist der Arbeitskanal möglichst vielseitig verwendbar und ist möglichst gerade bzw. weist nur relativ große Verbiegeradien auf.

Optional kann die distale Arbeitskanalöffnung distal von einer distalen Fluidkanalöffnung des Fluidkanals angeordnet sein. Damit kann durch den Arbeitskanal insbesondere ein zertrümmerter Nieren- oder Harnstein abgeführt werden, wobei durch den lateralen Ab- oder Auslauf von Spülungsflüssigkeit durch die Fluidkanalöffnung für klare Sichtverhältnisse gesorgt wird.

Optional kann der Querschnitt der distalen Arbeitskanalöffnung kleiner sein als der Querschnitt des Arbeitskanals. Damit wird das Verstopfungsrisiko des Arbeitskanals reduziert, da die kleinere Arbeitskanalöffnung wie ein Filter für zu große Gewebestücke wirkt und diese die Arbeitskanalöffnung nicht passieren können.

Optional kann sich der Querschnitt des Arbeitskanals zur distalen Arbeitskanalöffnung hin verjüngen. Damit wird eine innenseitige Stufe oder Kante vor der kleineren Arbeitskanalöffnung vermieden, vor welche ein durch den Arbeitskanal eingeschobener Laserlichtleiter, ein Fangkorbeinsatz oder ein Dorma-Schlingen-Einsatz stoßen könnte.

Optional kann eine Durchflussrichtung und/oder Durchflussrate durch den Fluidkanal wählbar oder einstellbar sein. Dies kann beispielsweise über eine Drucksteuerung und/oder durch Öffnen und Schließen eines Ventils, vorzugsweise an der proximalen Fluidkanalöffnung, durchgeführt werden.

Optional kann ein distales Schaftende steuerbar gelenklos abwinkelbar sein. Dies kann vorzugsweise wie oben beschrieben über eine durch seitliche Schlitze im Schaft lokal erhöhte Verbiegeflexibilität des Schafts und mindestens einen seitlich im distalen Schaftende angreifenden Seilzug realisiert sein.

Gemäß einem weiteren Aspekt der vorliegenden Offenbarung kann bei der Verwendung eines Lichtleiters und insbesondere Laserlichtleiters mit einem Außendurchmesser von deutlich unter 1 mm in einem Schaft eines endoskopischen Instruments mit gelenklos abwinkelbarer distaler Spitze einen dedizierten Arbeitskanal mit einem Innendurchmesser eingesetzt sein, der deutlich geringer als der Innendurchmesser eines herkömmlichen Arbeitskanals ist. Es ist bevorzugt, dass der Innendurchmesser eines Arbeitskanals für einen Lichtleiter den Außendurchmesser eines Lichtleiters um höchstens 30% überschreitet. Dadurch wird erlaubt, einen Lichtleiter auch bei abgewinkelter distaler Spitze des endoskopischen Instruments in ihrem Arbeitskanal verschieben zu können. Ein relativ scharfkantiges Ende des Lichtleiters erfährt durch das angegebene Durchmesserverhältnis auch bei gebogenem Verlauf des Arbeitskanals nur einen relativ geringen Angriffswinkel zu seiner Oberfläche und verletzt ihn nicht. Der Arbeitskanal und das endoskopische Instrument werden folglich nicht undicht und kann für eine weitere Dauer eingesetzt werden. Eine Spülleistung wird durch Ersetzen eines herkömmlichen Arbeitskanals durch einen deutlich schmaleren Arbeitskanal weiterhin verbessert. Der Außendurchmesser des Lichtleiters kann in einem Bereich von 0,4 mm bis 0,7 mm liegen, bevorzugt in einem Bereich von 0,45 mm bis 0,6 mm und besonders bevorzugt von 0,45 bis 0,5 mm. Es bietet sich besonders an, ein endoskopisches Instrument mit einem solchen separaten Arbeitskanal auszustatten, bei dem im Schaft ein Fluidkanal ausgebildet ist, der mindestens zwei durch den Schaft verlaufende Leitungen jeweils einzeln unmittelbar umgibt. Insbesondere bietet es sich an, diesen separaten Arbeitskanal in einem mit Schlitzen ausgestatteten distalen Bereich einzusetzen, um die Größe des darin gebildeten Fluidkanals noch weiter zu optimieren. Durch den geringen Durchmesser wird nur wenig Platz im Innenvolumen des Fluidkanals beansprucht. Wie vorangehend erwähnt kann der Lichtleiter insbesondere ein Laserlichtleiter sein.

Optional weist das Material des separaten Arbeitskanals für den Lichtleiter Polyimid oder Polyamid auf. Der Arbeitskanal für den Lichtleiter sollte aus einem möglichst harten Werkstoff bestehen und darf hierbei im gebogenen Zustand seinen Querschnitt nur möglichst wenig verändern. Dadurch wird die Gefahr, dass der Lichtleiter in die Innenfläche des Arbeitskanals eindringt, wesentlich verringert. Der Werkstoff für den Arbeitskanal kann etwa Polyimid oder Polyamid (auch als Nylon bekannt) in entsprechender Härte sein.

Optional kann die Wandung des separaten Arbeitskanals für den Lichtleiter Verstärkungen aufweisen. Diese können insbesondere in umlaufender Richtung verlaufen und ein Einknicken des Querschnitts des separaten Arbeitskanals verhindern.

Weiter optional kann das Material des separaten Arbeitskanals auch einen metallischen Werkstoff aufweisen. Dies kann etwa Nitinol umfassen, das eine Nickel-Titan-Legierung mit superelastischen Eigenschaften bei Raumtemperatur ist. Dieser Werkstoff kann bei den vorangehend genannten kleinen Durchmessern die geforderte und notwendige Elastizität/Flexibilität/Biegemöglichkeit aufweisen.

Gemäß einem weiteren Aspekt der vorliegenden Offenbarung kann bei der Verwendung eines endoskopischen Instruments mit einem Schaft mit abwinkelbarer distaler Spitze eine Anordnung von ersten Schlitzen vorgesehen sein, die sich optional abwechselnd auf einer ersten lateralen Seite des Schafts und einer diametral der ersten gegenüberliegenden zweiten lateralen Seite des Schafts befinden. Dadurch kann eine Abwinkelung in einer Hauptabwinkelungsebene erreicht werden, ohne einzelne, durch ein Gelenk verbundene Segmente schaffen zu müssen, die sich teilweise in das Innenlumen des Schafts erstrecken. Zur weiteren Verbesserung der Bewegbarkeit können sich optional proximalwärts an den Bereich mit ersten Schlitzen mehrere zweite Schlitze anschließen, die um 90° zu den ersten Schlitzen versetzt sind. Die zweiten Schlitze können sich in Umfangsrichtung bevorzugt nur über einen Teil des Schaftumfangs erstrecken. Weiter bevorzugt sind die zweiten Schlitze axial zueinander derart angeordnet, dass sie abwechselnd auf einer dritten lateralen Seite des Schafts und einer diametral der dritten gegenüberliegenden vierten lateralen Seite des Schafts liegen. Hierdurch wird eine zweite Abwinkelungsebene gebildet, die quer zu der Hauptabwinkelungsebene liegt.

Je nach Anzahl der zweiten Schlitze kann die Bewegbarkeit in der zweiten Abwinkelungsebene eingestellt werden. Es bietet sich besonders an, die Anzahl der zweiten Schlitze derart zu wählen, dass das distale Ende des Schafts in der zweiten Abwinkelungsebene um einen Winkelbereich von mindestens +/-15° und bevorzugt von +/-20° bewegbar ist. Damit kann insbesondere die Erreichbarkeit von Nierensteinen in unteren Kelchgruppen der Nieren verbessert werden.

Die Bewegung des distalen Endes des Schafts in der zweiten Abwinkelungsebene kann durch separate mechanische Leitungen, insbesondere Zugdrähte, erreicht werden. Diese sind direkt distal vor dem mit zweiten Schlitzen ausgestatteten Bereich mit dem Schaft gekoppelt. Zur Bewegung der Zugdrähte kann eine vorangehend genannte Handhabungseinrichtung eingesetzt werden.

Gemäß einem weiteren Aspekt der vorliegenden Offenbarung kann bei der Verwendung eines endoskopischen Instruments, das einen mit einer Handhabungsvorrichtung koppelbaren Schaft mit abwinkelbarer distaler Spitze aufweist und in dem ein Fluidkanal ausgebildet ist, der mindestens zwei durch den Schaft verlaufende Leitungen jeweils einzeln unmittelbar umgibt, eine Dichtungsvorrichtung bzw. ein Dichtmittel vorgesehen sein, um die Leitungen gegenüber der Handhabungsvorrichtung abzudichten. Das Dichtmittel bildet folglich ein proximales Ende des Fluidkanals. Bevorzugt ist das Dichtmittel drehfest mit dem Schaft gekoppelt. Das Verdrehen des Schafts führt folglich zu einer Verdrehung der Leitungen und des Dichtmittels, so dass die Relativpositionen der durch das Dichtmittel durchgeführten Leitungen zu dem Dichtmittel unverändert bleiben. Das Dichtmittel wird daher nicht durch Querkräfte belastet, die das Material des Dichtmittels elastisch so verformen könnten, dass eine Dichtwirkung zumindest bereichsweise aufgehoben wird. Die drehfeste Kopplung kann durch Formschlussmittel realisiert werden, die in dem Dichtmittel und in einer Dichtungsaufnahme miteinander korrespondierend ausgebildet sind.

Optional weist das Dichtmittel eine scheibenförmige, runde Form auf. Das Dichtmittel kann dabei leicht in eine Dichtungsaufnahme eingepresst werden.

Optional können die Formschlussmittel in Form einer umfangsseitigen Nut- und Federanordnung realisiert sein. Die Nut kann in eine Umfangsfläche des Dichtmittels ragen oder in der Dichtungsaufnahme ausgebildet sein. Die Feder könnte in der Dichtungsaufnahme oder in dem Dichtmittel als radialer Vorsprung ausgebildet sein. Bevorzugt sind die Formschlussmittel ausschließlich an einer einzigen, umfangsseitigen Position angeordnet, so dass auch bei unsymmetrisch gestaltetem Dichtmittel ein korrektes Platzieren des Dichtmittels erreicht wird.

Optional kann das Dichtmittel zumindest zwei Schlitze als Durchführungen aufweisen, die eine Umfangsfläche des Dichtmittels durchsetzen und sich jeweils teilweise durch das Dichtmittel erstrecken. Die Schlitze können dazu vorgesehen sein, mechanische Leitungen, wie etwa Zugdrähte, durch das Dichtmittel durchzuführen. Die Zugdrähte erstrecken sich von der Handhabungseinrichtung zu dem distalen Ende des Schafts und können dazu eingesetzt werden, die distale Spitze gelenklos abzuwinkeln. Werden die Zugdrähte als Flachdrähte ausgeführt, können auch diese zuverlässig abdichtend durch das Dichtmittel geführt werden.

Optional kann das Dichtmittel vier Schlitze als Durchführungen aufweisen, die voneinander beabstandet in dem Dichtmittel angeordnet sind. Das Dichtmittel kann folglich auch zum Abdichten eines Fluidkanals in einem Schaft eines endoskopischen Instruments eingesetzt werden, bei dem die Spitze in einer Hauptabwinkelungsebene abwinkelbar ist und zusätzlich in einer zweiten Abwinkelungsebene bewegbar ist. Das endoskopische Instrument kann folglich vier mechanische Leitungen in Form von Zugdrähten oder dergleichen aufweisen, die durch die vier Schlitze durchgeführt werden und sich durch den Schaft erstrecken. Die Größe und Ausrichtung der Schlitze ist dabei von der Art und Anordnung der Zugdrähte abhängig.

Weiter optional können die Schlitze auf parallelverschobenen Radiallinien basieren, die um mindestens 90° zueinander auf einer von dem Dichtmittel überdeckten Fläche versetzt sind. Die Schlitze können über eine senkrecht zu dem jeweiligen Schlitz verlaufende, einen Mittelpunkt des Dichtmittels schneidende Mittellinie hinausragen. Werden vier Zugdrähte eingesetzt, sind vier Schlitze erforderlich, welche dann beispielsweise um 90° zueinander versetzt sind. Bei lediglich zwei Zugdrähten könnten zwei Schlitze ausreichen, die dann beispielhaft um 180° zueinander versetzt sind.

Optional kann das Dichtmittel aus einem Material hergestellt sein, das Polyethylen oder Zellkautschuk aufweist. Zellkautschuk kann beispielsweise auf einem Fluorpolymer basieren.

Optional kann das endoskopische Instrument, welches das vorangehend dargestellte Dichtmittel aufweist, ein drehbar in einem Steuergehäuse aufweisendes Schaftanschlussteil aufweisen, das mit dem Schaft fest verbindbar ist. Das Schaftanschlussteil kann optional einen Anschlussstutzen aufweisen, der sich radial von einer Schaftanschlussachse nach au-βen erstreckt. Zwischen einer Dichtungsaufnahme zum Aufnehmen des Dichtmittels und dem Schaftanschlussteil kann optional ein Dichtring angeordnet sein, um eine Abdichtung zu erreichen.

Weiter optional weist das Schaftanschlussteil Führungseinrichtungen zum Führen von Zugdrähten auf. Durch Führen der Zugdrähte können diese unmittelbar jeder Verdrehung des Schaftanschlussteils folgen. Ein Verschränken der Zugdrähte und der zugehörigen Schlitze in dem Dichtmittel kann verhindert werden, so dass die Dichtwirkung dadurch nicht beeinträchtigt wird. Die Dichtungsaufnahme kann weiterhin mit einem von dem Schaftanschlussteil abgewandten Deckel in distaler Richtung ergänzt werden, wobei der Deckel ebenso drehfest mit dem Schaftanschlussteil gekoppelt ist. Der Deckel kann weiterhin ebenso Führungseinrichtungen aufweisen, mit denen Zugdrähte geführt werden können. Die Führungseinrichtungen in dem Deckel und in dem Schaftanschlussteil fluchten bevorzugt, so dass das Dichtmittel ausschließlich Axialkräfte der Zugdrähte erfährt.

Gemäß einem weiteren Aspekt der vorliegenden Offenbarung kann bei einem endoskopischen Instrument, das einen mit einer Handhabungsvorrichtung koppelbaren Schaft aufweist, ein Schaftwerkzeug, beispielsweise mit einem darin integrierten Fangkorb oder einer Zange, durch einen Arbeitskanal des Instruments an das distale Ende des Instruments geschoben werden. Die Handhabungsvorrichtung kann hierzu bevorzugt einen Arbeitskanaleinlass, ein erstes drehbares Aufnahmeteil und ein zweites drehfest mit dem ersten Aufnahmeteil verbundenes zweites Aufnahmeteil aufweisen. Das erste Aufnahmeteil kann fest mit einem Mantelelement des Schaftwerkzeugs koppelbar sein, beispielsweise über einen Luer-Anschluss, gleichwohl zur genauen axialen Positionierung relativ zu dem Arbeitskanaleinlass verschiebbar gelagert sein. Das zweite Aufnahmeteil kann über einen durch das Mantelelement des Schaftwerkzeugs geführten Steuerdraht mit einem distalen Werkzeugkopf, beispielsweise einem Fangkorb oder Zangenmaulteilen, gekoppelt und relativ zu dem ersten Aufnahmeteil axial verschiebbar gelagert sein. Durch das erste Aufnahmeteil kann das Schaftwerkzeug in unterschiedliche axiale Positionen im Instrument geschoben werden. Damit ist es möglich, das Schaftwerkzeug an die distale Schaftspitze positioniert durch den Schaft einzuschieben, sodass es zunächst nicht über sie hinausragt. Die distale Schaftspitze kann folglich ungestört zu einem Operationsbereich bewegt werden und eine Verletzungsgefahr durch einen scharfkantigen Werkzeugkopfwird vermieden. Nach dem Erreichen des Operationsbereichs kann durch Verschieben des ersten Aufnahmeteils in distaler Richtung das Schaftwerkzeug aus dem distalen Schaftende herausgeschoben werden. Der Draht, der mit dem Werkzeugkopf, beispielsweise dem Fangkorb oder Zangenmaulteilen, gekoppelt ist, kann ggf. anschließend durch Verschieben des zweiten Aufnahmeteils aus dem Mantelelement des Schaftwerkzeugs herausgeschoben werden. Durch eine drehfeste Kopplung des Schaftwerkzeugs und des ersten Aufnahmeteils sowie eine drehfeste Kopplung zwischen dem zweiten Aufnahmeteil und dem ersten Aufnahmeteil kann das Schaftwerkzeug verdreht werden. Durch diesen Aufbau ist es zudem sehr leicht möglich, das Schaftwerkzeug durch einen einzigen Benutzer zu positionieren und zu betätigen. Eine Abstimmung mit einem zweiten Benutzer ist folglich nicht erforderlich.

Das erste Aufnahmeteil ist optional um einen ersten Verschiebeweg gegenüber dem Arbeitskanaleinlass verschiebbar. Das zweite Aufnahmeteil ist über einen zweiten Verschiebeweg relativ zu dem ersten Aufnahmeteil bewegbar. Die Länge des zweiten Verschiebewegs kann dabei optional die Länge des ersten Verschiebewegs über- oder unterschreiten. Der erste Verschiebeweg ist ausschließlich dazu gedacht, das Schaftwerkzeug in eine axial außerhalb der distalen Schaftspitze liegende Position zu bringen. Damit kann der Werkzeugkopf störungsfrei aus der distalen Spitze herausgefahren werden. Die Länge des ersten Verschiebewegs könnte lediglich wenige Millimeter betragen. Es ist denkbar, einen Verschiebeweg von ungefähr 5 mm vorzusehen. Der erste Verschiebeweg kann je nach Ausführung des endoskopischen Instruments auch in einem Bereich von 2 mm bis 10 mm liegen. Der zweite Verschiebeweg ist indes von der Art und Grö-βe des Werkzeugkopfs abhängig und kann beispielhaft bis zu 20 oder 25 mm betragen.

Das erste Aufnahmeteil und/oder das zweite Aufnahmeteil können optional über Rastmittel in einer momentanen axialen und/oder gedrehten Position gehalten werden. Das erste Aufnahmeteil und/oder das zweite Aufnahmeteil könnten beispielsweise eine Mantelfläche aufweisen, die mit parallel zueinander angeordneten, umlaufenden Rillen ausgestattet ist, wobei die Rillen jeweils einen abgerundeten Profilquerschnitt aufweisen. Werden diese durch eine entsprechend dimensionierte Öffnung geführt, kann das Einrasten zwischen zwei aufeinanderfolgenden Rillen erfolgen. Durch entsprechende Kraft quer zu den Rillen kann die eingerastete Position allerdings auch wieder gelöst werden.

Das erste Aufnahmeteil und/oder das zweite Aufnahmeteil können ferner mit einem Absatz ausgestattet sein, der jeweils eine Rändelung aufweist. Dadurch kann ein Benutzer das betreffende Aufnahmeteil gut greifen und gefühlvoll und mit direkter, haptischer Rückkopplung eine Verdrehung durchführen.

Gemäß einem weiteren Aspekt der vorliegenden Offenbarung kann die Handhabungseinrichtung eines endoskopischen Instruments, das mit einem Schaft ausgestattet ist, ein Gehäuse aufweisen, das an einem proximalen Ende zwei Schenkel besitzt, die schräg zueinander angeordnet sind und eine Anlagefläche einschließen, wobei mindestens eine Leitung aus einem der Schenkel aus der Handhabungseinrichtung nach außen geführt ist. Die Handhabungseinrichtung kann folglich an einem proximalen Ende wie ein Y oder ein V ausgebildet sein. Einem Benutzer wird ermöglicht, die Handhabungseinrichtung so zu greifen, dass ein Daumenballen einer Hand auf der Anlagefläche aufliegt und zwei weitere Finger der Hand, beispielsweise Zeigefinger und Mittelfinger, zu Abzügen bzw. Bedienhebeln geführt werden können, die distalwärts angeordnet sind. Das Gehäuse ist derart ausgebildet, dass der Daumenballen so auf der Anlagefläche liegen kann, dass die Handhabungseinrichtung eine direkte Verlängerung des Unterarms des Benutzers bildet. Ein Verdrehen des Unterarms führt zu einer direkten und ausschließlichen Verdrehung der Handhabungseinrichtung um die Längsachse des Schafts, sodass hierdurch auch der daran angebrachte Schaft um seine Längsachse verdreht wird.

Distalwärts im Anschluss an die beiden Schenkel kann optional mindestens eine Steuerscheibe folgen, die vorzugsweise radialseitig mit Zugdrähten und mindestens zwei Abzügen oder Bedienhebeln zum Bewegen der mindestens einen Steuerscheibe gekoppelt ist. Die Steuerscheibe ist vorzugsweise um eine Drehachse im Gehäuse der Handhabungseinrichtung in einem Winkelbereich vorzugsweise in zwei Richtungen drehbar, wobei die Drehachse vorzugsweise im Wesentlichen senkrecht zu einer von den Schenkeln aufgespannten Ebene verläuft.

Die mindestens eine Steuerscheibe ist optional über eine Arretiereinrichtung in ihrer momentanen Position fixierbar. Die Arretiereinrichtung könnte beispielsweise in Form einer Rändelschraube realisiert sein, mit der ein Benutzer eine einmal eingestellte Position der mindestens einen Steuerscheibe arretieren kann. Eine durch die Zugdrähte hervorgerufene Abwinkelung kann folglich in ihrem Zustand verbleiben, ohne dass sie aufwändig durch dauerhaftes manuelles Einwirken beibehalten werden muss.

Die zuvor beschriebenen Aspekte der vorliegenden Offenbarung finden zwar bevorzugt in beliebiger Kombination in den Ausführungsformen eines hierin offenbarten endoskopischen Instruments Anwendung, können allerdings auch jeweils unabhängig voneinander ohne die anderen Aspekte vorteilhaft eingesetzt werden. Eine Vorrichtung gemäß dem` Oberbegriff des unabhängigen Anspruchs 1 ist aus der Druckschrift US 2016/100745 A1 bekannt. Die Erfindung selbst ist im unabhängigen Anspruch 1 definiert.

Nachfolgend ist die Erfindung anhand von in den Zeichnungen dargestellten Ausführungsbeispielen näher erläutert. Es zeigen:
- Fig. 1: eine perspektivische Ansicht eines Beispiels einer Ausführungsform eines hierin offenbarten endoskopischen Instruments;
- Fig. 2a,b: perspektivische Ansichten eines distalen Endabschnitts einer beispielhaften Ausführungsform eines hierin offenbarten endoskopischen Instruments;
- Fig. 3a-c: Explosionsansichten Ansicht von Teilen einer beispielhaften Ausführungsform eines hierin offenbarten endoskopischen Instruments;
- Fig. 4a,b: schematische Querschnittsansichten eines distalen Endabschnitts eines Arbeitskanals einer beispielhaften Ausführungsform eines hierin offenbarten endoskopischen Instruments;
- Fig. 5: eine Querschnittsansicht eines proximalen Anschlussabschnitts einer beispielhaften Ausführungsform eines hierin offenbarten endoskopischen Instruments;
- Fig. 6a-c: Seitenansichten einer Handhabungseinrichtung mit Detailansichten des distalen Schaftendes einer beispielhaften Ausführungsform eines hierin offenbarten endoskopischen Instruments;
- Fig. 7a-f: verschiedene Ansichten auf dem Schaft und insbesondere das distale Schaftende einer beispielhaften Ausführungsform eines hierin offenbarten endoskopischen Instruments;
- Fig. 8a-e: weitere Ansichten auf dem Schaft und insbesondere einen krümmbaren Schaftabschnitt einer beispielhaften Ausführungsform eines hierin offenbarten endoskopischen Instruments;
- Fig. 9a-d: verschiedene Ansichten auf ein Ausführungsbeispiel eines hierin offenbarten endoskopischen Instruments; und
- Fig. 10a-c: weitere Ansichten auf das Ausführungsbeispiel gemäß Fig. 9a-d.

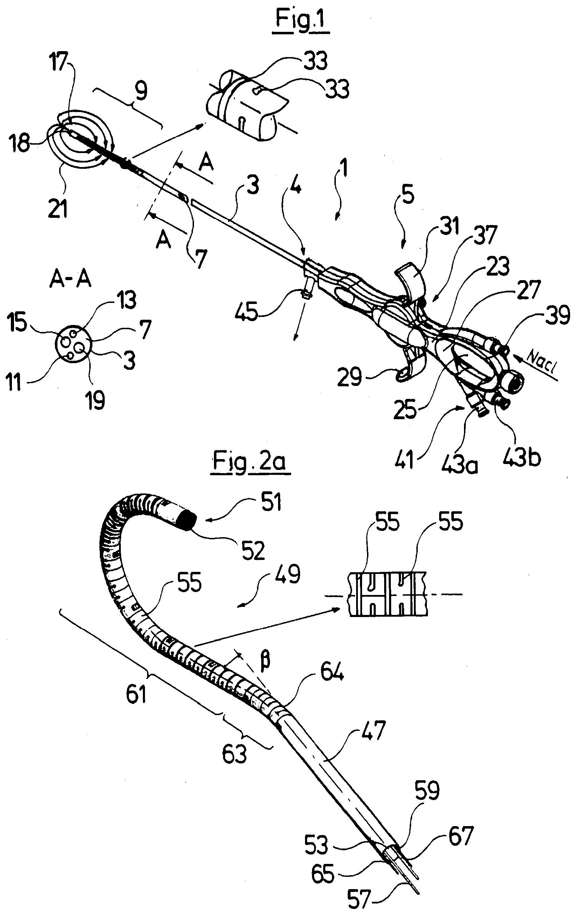

Fig. 1 zeigt beispielhaft ein endoskopisches Instrument 1, das einen rohrförmigen Schaft 3 zum Einführen in eine Körperhöhle aufweist. Der Schaft 3 ist an einem proximalen Ende 4 mit einer Handhabungseinrichtung 5 verbunden und bildet in seinem Innenlumen einen Fluidkanal 7 aus. An einem distalen krümmbaren Schaftabschnitt9 ist der Schaft3 weiterhin zumindest teilweise flexibel ausgebildet, um bedarfsweise durch Einwirkung der Handhabungseinrichtung 5 gekrümmt zu werden.

In einem Teilschnitt A-A durch den Schaft 3 ist ersichtlich, dass in dem Fluidkanal 7 beispielhaft eine erste optische und/oder elektrische Leitung 11 für die Beleuchtung am distalen Schaftende 17, eine zweite optische und/oder elektrische Leitung 13 für die Bildaufnahme am distalen Schaftende 17, ein erster Arbeitskanal 15 und ein zweiter Arbeitskanal 19 verlaufen. Am distalen Ende der ersten Leitung 11 kann eine LED als Lichtquelle angeordnet sein, wobei die Leitung 11 als elektrische Stromversorgungsleitung der LED dient. Alternativ oder zusätzlich kann am distalen Ende der ersten Leitung 11 eine Lichtleiterauskopplung als Lichtquelle angeordnet sein, wobei die Leitung 11 als optischer Lichtleiter dient. Analog dazu kann am distalen Ende der zweiten Leitung 13 kann ein Objektiv mit Bildsensor für die Bildaufnahme angeordnet sein, wobei die Leitung 11 als elektrische Stromversorgungsleitung des Bildsensors und zur Signalübertragung dient. Alternativ oder zusätzlich kann am distalen Ende der zweiten Leitung 13 eine Lichtleitereinkopplung angeordnet sein, wobei die Leitung 13 als optischer Lichtleiter dient.

Der erste Arbeitskanal 15 hat vorzugsweise einen mindesten doppelt so großen Querschnitt wie der zweite Arbeitskanal 19. Der erste Arbeitskanal 15 kann wahlweise als Einschubkanal für ein Schaftwerkzeug, beispielsweise einen Fangkorb, eine Schere oder Zange, dienen. Alternativ oder zusätzlich, selbst bei eingeschobenem Schaftwerkzeug, kann der erste Arbeitskanal 15 als Zu- oder Abführleitung von Spülflüssigkeit dienen. Der zweite Arbeitskanal 19 kann vorzugsweis als Einschubkanal für einen Laserlichtleiter dienen, um mit Laserlicht Nieren- oder Harnsteine zertrümmern zu können. Der Innendurchmesser des zweiten, kleineren Arbeitskanals 19 sollte zumindest im krümmbaren Schaftabschnitt 9 den Außendurchmesser des Laserlichtleiters um höchstens 30% überschreiten, um eine sichere Durchführung des Laserlichtleiters zu gewährleisten.

Sämtliche Leitungen 11, 13, 15, und 19 sind einzeln unmittelbar durch den Fluidkanal 7 umgeben. Die Besonderheit dieser Anordnung liegt darin, dass die vier exemplarischen Leitungen 11, 13, 15 und 19 einzeln in dem Fluidkanal 7 angeordnet sind und, wenn dort ein Fluid strömt, folglich unmittelbar durch das Fluid umspült werden. Die gesamte freie Restquerschnittsfläche des Fluidkanals 7 kann folglich für ein Fluid verwendet werden. Die Querschnittsfläche des Fluidkanals 7 entspricht folglich der Querschnittsfläche eines vom Schaft 3 gebildeten Schaftinnenraums abzüglich der Summe der Querschnittsflächen aller durch den Schaftinnenraum verlaufenden Leitungen 11, 13, 15 und 19 sowie aller weiteren Leitungen, die in Erwägung gezogen werden. Ein separater Fluidkanal, der stets eine eigenständige Ummantelung innerhalb des Schafts 3 erfordert, ist nicht notwendig, denn der erste Arbeitskanal 15 kann selbst bei eingeschobenem Schaftwerkzeug als Zu- oder Abführleitung von Spülflüssigkeit verwendet werden. Der Schaft 3 kann folglich mit einem besonders geringen Außendurchmesser versehen werden. Das etwaige Problem einer erschwerten Reinigbarkeit bzw. Sterilisierbarkeit kann bei diesem Aufbau dadurch gelöst werden, dass das ganze endoskopische Instrument 1 vorzugsweise als Verbrauchsartikel zum Entsorgen nach einmaliger Verwendung ausgestaltet ist, also gar nicht erst nach Gebrauch sterilisiert werden muss.

Selbstverständlich sind auch andere, weniger oder mehr Leitungen denkbar, die auf diese Weise in dem Fluidkanal 7 verlaufen können. Es sind etwa Leitungen zum Übertragen von elektrischen Signalen und/oder von elektrischer Leistung denkbar. Hierüber können etwa distal angeordnete Leuchtdioden mit elektrischer Leistung versehen werden oder Bildsignale können von einem distal angeordneten Bildsensor übertragen werden. Die Leitungen 11, 13, 15 und 19 sowie alle alternativen oder zusätzlichen Leitungen können jeweils eine eigene Ummantelung aufweisen. Dies kann insbesondere im Fall elektrischer Leitungen wichtig sein, um diese zu isolieren. Zusätzlich können mechanischen Leitungen in Form von Steuerseilen im Schaft geführt sein, den krümmbaren Schaftschnitt 9 gelenklos abzuwinkeln oder auf andere Weise in seiner Form zu steuern. Die mechanischen Leitungen können etwa an lateralen Innenflächen im Schaft 3 geführte Seilzüge bzw. Zugdrähte sein.

Der distale krümmbare Schaftabschnitt 9 als distaler Bereich ist mit mehreren Schlitzen 33 versehen, die dem krümmbare Schaftabschnitt 9 seine Beweglichkeit verleihen. Der distale krümmbare Schaftabschnitt 9 ist folglich flexibel oder zumindest teilweise flexibel, also semi-flexibel. Statt der Verwendung einzelner, gelenkig miteinander verbundener Glieder kann der Schaftabschnitt 9 durch gezieltes Schlitzen flexibel um zumindest eine Krümmungsachse biegbar gemacht werden. Die Schlitze sind hierzu bevorzugt in Umfangsrichtung in den Schaft 3 eingebracht, erstrecken sich über die gesamte Materialdicke des Schaftmantels und verlaufen über mehr als der Hälfte des Umfangs, beispielsweise um bis zu 270°. Zwei entlang der Erstreckungsrichtung des Schafts 3 aufeinanderfolgende lamellenartige Teile des Schafts 3, zwischen denen sich ein Schlitz 33 in einer Umfangsfläche 35 des Schafts 3 befindet, sind folglich durch einen Steg miteinander verbunden, der sich in diesem Fall über beispielhaft mindestens 90° in Umfangsrichtung erstreckt. Aufgrund der einstückigen Ausgestaltung sind die zwischen den einzelnen lamellenartigen Teilen verbleibenden Stege stets bestrebt, eine ursprüngliche, unbelastete Position einzunehmen, in der der Schaft 3 bevorzugt geradlinig verläuft. Wird der vorzugsweise metallische Schaft 3 folglich durch Einwirkung einer Zugkraft durch ein Zugseil abgewinkelt, kann der distale krümmbare Schaftabschnitt 9 um bis zu 300° gelenklos abgewinkelt werden. Der Schaft 3 ist nach Lösen der Zugkraft durch die zurückfedernden Stege bestrebt, wieder in seine gerade Form zurückzukehren. Um eine gleichmäßige Flexibilität zu erreichen sind die Schlitze 33 axial derart zueinander angeordnet, dass sie abwechselnd auf einer ersten lateralen Seite des Schafts 3 und einer diametral der ersten gegenüberliegenden zweiten lateralen Seite des Schafts 3 liegen.

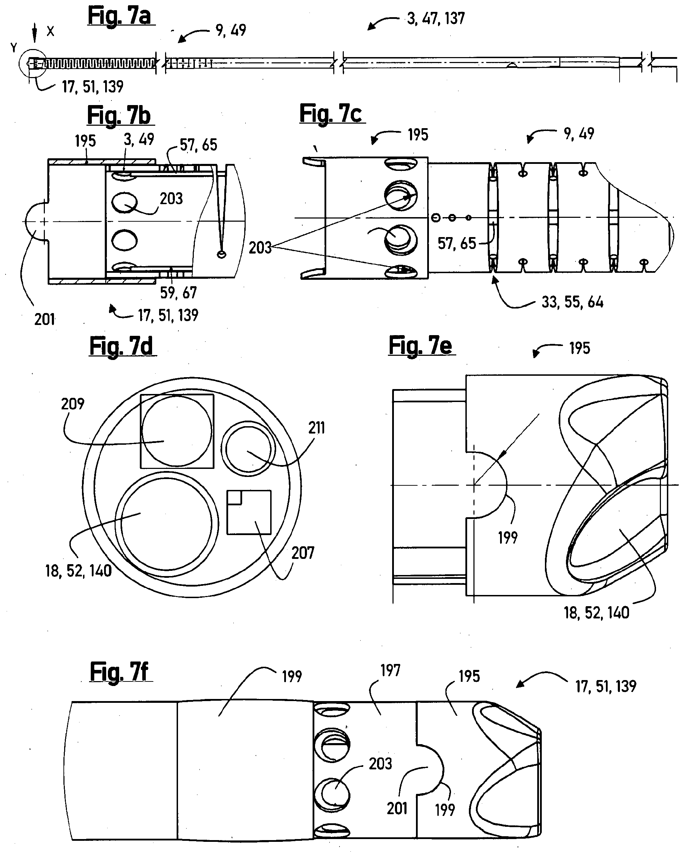

Die Schlitze 33 können durch eine Bearbeitungsvorrichtung in den Schaft 3 eingebracht werden, die bevorzugt einen Laser einsetzt. Dadurch kann eine rasche, kostengünstige Bearbeitung erfolgen, die einer Ausgestaltung des Instruments 1 als Verbrauchsartikel nicht entgegensteht. Durch eine geeignete Strahlführung des Lasers können sehr filigrane Strukturen hergestellt werden. Die lichte Weite der Schlitze 33 kann so klein dimensioniert werden, dass Gewebe nicht in die Schlitze 33 eindringt und ein ungestörtes Verschieben des Schafts 3 in den Operationsbereich erfolgen kann. Die Schlitze 33 können in ihrem Profil abgerundet sein. Optional kann eine Beschichtung des Schafts 3 mit einer Gleitbeschichtung erfolgen. Der geschlitzte, krümmbare Schaftabschnitt 9 ist vorzugsweise durch einen Schutzschlauch 199 (siehe Fig. 7f) umgeben. Der Schutzschlauch kann beispielsweise gewebeverstärkt und/oder aufgeschrumpft sein. Der Schutzschlauch kann beispielsweise biokompatiblen Kunststoff aufweisen.

Der Fluidkanal 7 im Innenlumen des Schafts 3 kann als ein Zu- und/oder Abführkanal für eine Spülflüssigkeit dienen, die durch den freien Restquerschnitt gefördert wird. Es ist vorstellbar, dass nach Bedarf Spülflüssigkeit zum Verbessern der Sicht an dem distalen Ende 17 distalwärts aus den Schlitzen 33, die dann als distale Fluidkanalöffnungen wirken, zugeführt wird oder Spülflüssigkeit mit störender Gewebesuspension proximalwärts abgeführt wird. Bei ausschließlicher Verwendung des Fluidkanals 7 ohne den ersten Arbeitskanal 15 als Zu- und/oder Abführkanal ist vorstellbar, Spülflüssigkeit zeitweise entweder nur zuzuführen oder nur abzuführen. Das Zuführen von Spülflüssigkeit kann beispielsweise aktiv durch eine externe Pumpe oder passiv durch hydraulischen Druck aus einem höher aufgehängten angeschlossenen Tropf erfolgen. Das Abführen kann erfolgen, indem durch das Spülen ein Überdruck in dem Operationsbereich generiert wird, durch den Spülflüssigkeit anschließend wieder proximalwärts außenseitig am Schaft 3 entlang austreten kann. Vorzugsweise dient jedoch der erste Arbeitskanal 15 als Zuführkanal und der Fluidkanal 7 als Abführkanal, oder umgekehrt. Dadurch kann ein unerwünschter Fluidfluss außenseitig am Schaft 3 weitgehend reduziert oder ganz vermieden werden.

Es kann also für die kontinuierliche Spülung sinnvoll sein, den ersten Arbeitskanal 15, der sich durch den Fluidkanal 7 distalwärts erstreckt und eine erste distale Arbeitskanalöffnung 18 aufweist, als Zu- und/oder Abführkanal zu verwenden. Der Querschnitt der ersten distalen Arbeitskanalöffnung 18 kann kleiner sein als der Querschnitt des ersten Arbeitskanals 15, so dass das Verstopfen des ersten Arbeitskanals 15 durch Gewebe verhindert wird, welches über die distale Arbeitskanalöffnung 18 eintritt. Alternativ oder zusätzlich dazu kann sich der Querschnitt des ersten Arbeitskanals 15 zu der distalen Arbeitskanalöffnung 18 hin verjüngen. Der erste Arbeitskanal 15 kann durch eine externe oder in der Handhabungseinrichtung 5 angeordnete Pumpe bzw. aus einem höher aufgehängten angeschlossenen Tropf kontinuierlich mit Spülflüssigkeit versorgt werden, die distalwärts geleitet wird und direkt an dem distalen Schaftende 17 austritt. Der erste Arbeitskanal 15 ist folglich ein Zuführkanal. Durch den sich einstellenden Überdruck gelangt die Spülflüssigkeit durch die Schlitze 33 und/oder laterale Spülöffnungen 203 (siehe Fig. 7b,c) in den Fluidkanal 7 und strömt von dort proximalwärts zurück. Dies wird in Fig. 1 mit den Strömungslinien 21 angedeutet, die aus einer rein axialen, distalwärts gerichteten Ausströmung aus dem distalen Schaftende 17 zu einer lateralen Einströmung durch die Schlitze 33 und/oder laterale Spülöffnungen führen. Die Strömungsrichtung in dem ersten Arbeitskanal 15 ist folglich gegenläufig zu der des Fluidkanals 7. Selbstverständlich kann dies auch in umgekehrter Konstellation erfolgen, indem Spülflüssigkeit aus den Schlitzen 33 und/oder den laterale Spülöffnungen in dem distalen krümmbaren Schaftabschnitt 9 austritt und an dem distalen Schaftende 17 über den ersten Arbeitskanal 15 wieder zurückgeführt wird. Falls die Schlitze 33 von einem Schutzschlauch 199 und/oder einer Gleitbeschichtung umgeben sind, kann die Fluidführung ausschließlich über laterale Spülöffnungen 203 erfolgen.

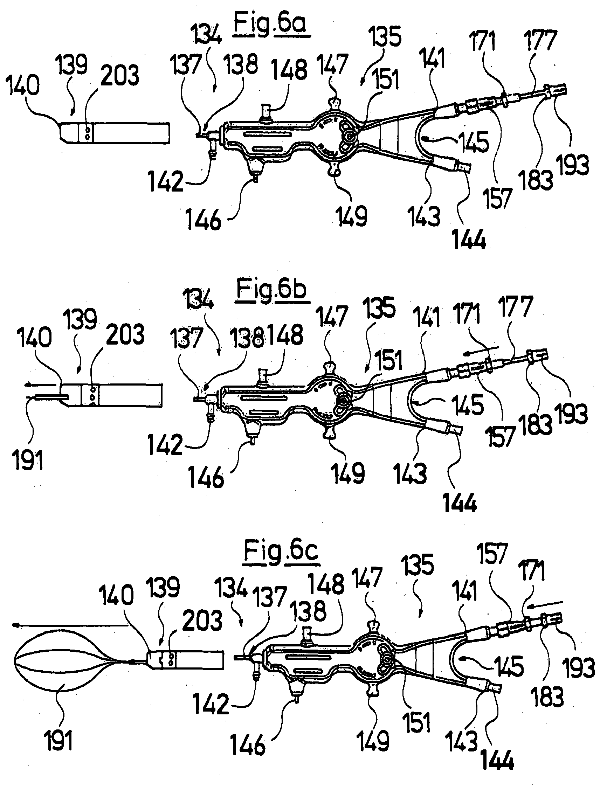

Die Handhabungseinrichtung 5 ist in Fig. 1 lediglich exemplarisch dargestellt. Weitere Handhabungseinrichtungen werden in den Figuren 6a-c ff. gezeigt. Sämtliche Varianten von Handhabungseinrichtungen können mit den hier geschilderten vorteilhaften Ausgestaltungen des Schafts 3 kombiniert werden. Es sind weiterhin auch andere Handhabungseinrichtungen denkbar, die in ihrer Form von den hierin gezeigten Varianten abweichen.

Bei der Handhabungseinrichtung 5 in Fig. 1 ist ein Gehäuse 23 gezeigt, das beispielsweise eine Grifföffnung 25 zum Durchführen eines Daumens besitzt und so ausgestaltet ist, dass der Daumenballen einer Hand dann auf einer zu dem Schaft 3 gerichteten Auflagefläche 27 aufliegt. Unmittelbar in Griffweite der anderen Finger der Hand schließen sich zwei an einander entgegengesetzten lateralen Seiten des Gehäuses 23 angeordnete Bedienhebel bzw. Abzüge 29, 31 an. Befindet sich der Daumenballen an der Auflagefläche 27, kann etwa der untere Abzug 29 mit einem Mittelfinger bedient werden, während ein Zeigefinger auf dem oberen Abzug 31 liegt. Durch Ziehen der Abzüge 29 und 31 zu der Grifföffnung 25 hin werden die damit gekoppelten Seilzüge 11 und 13 gezogen. Um Verspannungen innerhalb des Schafts 3 zu vermeiden und eine harmonische Bewegung des distalen krümmbaren Schaftabschnitts 9 durchzuführen, könnten die beiden Seilzüge jeweils als ein Abschnitt eines einzelnen Seilzugs ausgebildet sein, der über eine Führungsrolle zwischen den beiden Abzügen 29, 31 verläuft. Wird also etwa der untere Abzug 29 bewegt, könnte beispielsweise der erste Seilzug 11 gezogen werden, wobei der obere Abzug 31 in die entgegengesetzte Richtung bewegt wird und dabei den zweiten Seilzug 13 entspannt. Hierzu könnten beide Abzüge 29, 31 miteinander über die hier nicht gezeigte Führungsrolle verbunden oder mittelbar gekoppelt sein, sodass sie sich bei Betätigung selbstständig gegenläufig bewegen.

Der erste, größere Arbeitskanal 15 kann an einer zu dem oberen Abzug 31 gewandten Seite 37 der Handhabungseinrichtung 5 verlaufen, sodass dort eine erste proximale Arbeitskanalöffnung 39 zum wahlweisen Einführen einer Spülflüssigkeit, beispielsweise NaCI-Lösung, oder eines Schaftwerkzeugs vorliegt. Andere Leitungen, beispielsweise der zweite, kleinere Arbeitskanal 19 für einen Laserlichtleiter, können an einer zu dem unteren Abzug 29 gewandten Seite 41 in einem entsprechenden zweiten proximalen zweiten proximalen Arbeitskanalöffnung zweiten proximalen Arbeitskanalöffnung 43a oder 43b münden. Der Fluidkanal 7 weist eine proximale Fluidkanalöffnung 45 in Form eines quer zu dem Schaft 3 verlaufenden Stutzens auf, aus der die zurückströmende Spülflüssigkeit dann schließlich austritt.

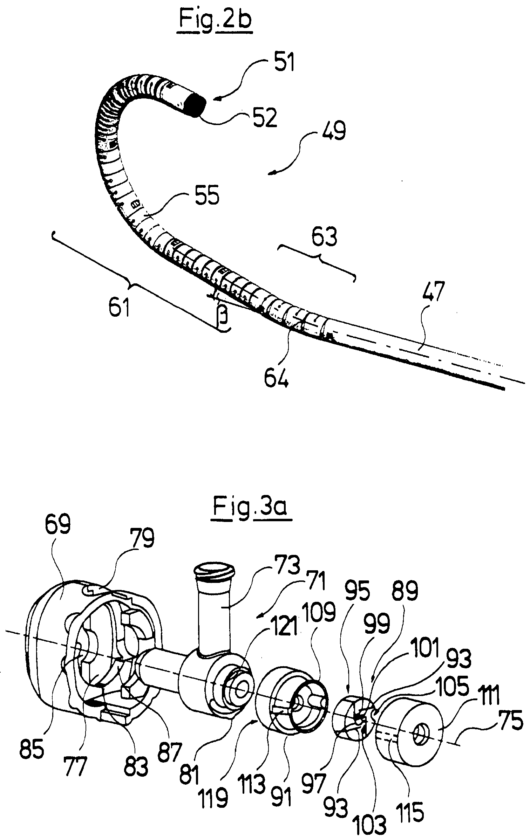

In den Fig. 2a und 2b wird ein Schaft 47 eines endoskopischen Instruments in einer Teilansicht dargestellt. Die Ansicht konzentriert sich auf einen flexiblen distalen krümmbaren Schaftabschnitt 49 mit einem distalen Ende 51 des Schafts 3, an dem eine erste distale Arbeitskanalöffnung 52 angeordnet ist. Der Querschnitt dieser ersten distalen Arbeitskanalöffnung 52 kann geringer sein als der des hier nicht gezeigten ersten Arbeitskanals 15. Alternativ oder zusätzlich dazu kann sich der Querschnitt des ersten Arbeitskanals 15 zu der ersten distalen Arbeitskanalöffnung 52 verjüngen. Der Schaft 47 weist proximal einen hier nicht sichtbaren, starren, rohrförmigen Abschnitt auf, der mit einer Handhabungseinrichtung 5 verbindbar ist. Diese kann der Handhabungseinrichtung 5 aus der Fig. 1 entsprechen oder wie eine in Fig. 6a-c dargestellte Handhabungseinrichtung ausgeführt sein.

Mit distalwärtiger Blickrichtung ist ein in dem Schaft 47 ausgebildeter Fluidkanal 53 erkennbar. Der distale krümmbare Schaftabschnitt 49 ist so ausgebildet, dass er auf möglichst einfachem Wege abgewinkelt werden kann, ohne dedizierte Gelenke zu erfordern, die in den Fluidkanal 53 hineinragen oder zu einem vergrößerten Außendurchmesser des Schafts 47 führen. Hierzu ist der distale krümmbare Schaftabschnitt 49 als distaler Bereich mit mehreren, parallel zueinander verlaufenden und voneinander beabstandeten ersten Schlitzen 55 versehen, die das Material des Schafts 47 lokal vollständig durchdringen. Die ersten Schlitze 55 erstrecken sich beispielhaft über einen Winkel von 270° in Umfangsrichtung und umlaufen dabei eine Haupterstreckungsrichtung des Schafts 47, die von einem proximalen Ende zu dem distalen Ende 51 hin verläuft. Beispielhaft sind aufeinander folgende bzw. benachbarte Schlitze 55 um 180° in Umfangsrichtung zueinander versetzt. Die ersten Schlitze 55 sind demnach axial derart zueinander angeordnet sind, dass sie abwechselnd auf einer ersten lateralen Seite des Schafts 47 und einer diametral der ersten gegenüberliegenden zweiten lateralen Seite des Schafts 47 liegen. Folglich erfährt der distale krümmbare Schaftabschnitt 49 dadurch eine sehr flexible Form. Einzelne, durch die Schlitze 55 voneinander getrennte lamellenartige Teile des Schafts 47 können durch Aufweiten bzw. Komprimieren der Schlitze 55 ihre Ausrichtung zueinander ändern. Dadurch erfährt der krümmbare Schaftabschnitt 49 eine gelenklose Abwinklung von bis zu 300°. Diese Bewegung des krümmbaren Schaftabschnitts 49 kann durch mechanische Zugdrähte 57 und 59 beeinflusst werden, die in dem Fluidkanal 53 verlaufen. Damit kann gezielt ein Ziehen und Entlasten lateraler Abschnitte des krümmbaren Schaftabschnitts 49 erfolgen.

Der gesamte Schaft 47 kann durch diese vorteilhafte Ausführung einteilig ausgestaltet sein, was sich besonders positiv auf den erreichbaren, minimalen Außendurchmesser des Schafts 47 auswirkt. Wie in der Darstellung der Fig. 1 sind auch hier zwei entlang der Erstreckungsrichtung des Schafts 47 aufeinanderfolgende lamellenartige Teile, zwischen denen sich ein Schlitz 55 befindet, folglich durch einen Steg miteinander verbunden, der sich in diesem Fall über 270° in Umfangsrichtung erstreckt. Aufgrund der einstückigen Ausgestaltung sind die zwischen den einzelnen Teilen verbleibenden Stege dazu bestrebt, eine ursprüngliche unbelastete Position einzunehmen, in der der Schaft 47 bevorzugt geradlinig verläuft. Wird der Schaft 47 folglich durch Einwirkung einer Zugkraft durch eine der mechanischen Leitungen 57 oder 59 abgewinkelt, ist der Schaft 47 nach Lösen der Zugkraft durch die zurückfedernden Stege bestrebt, in eine gerade Form zurückzukehren. Der krümmbare Schaftabschnitt 49 wirkt daher wie eine Feder. Ein den krümmbaren Schaftabschnitt 49 umgebender Schutzschlauch kann diese in die ursprünglich gerade Form hin drängende Federwirkung unterstützen.

Durch diese Anordnung der ersten Schlitze 55 kann eine Abwinkelung auf einer einzigen Ebene ausgeführt werden, die durch die umfangsseitigen Mittelpunkte der Schlitze 55 aufgespannt wird. Bei der speziellen Anwendung im Bereich der Zerstörung von Nierensteinen ist der distale krümmbare Schaftabschnitt 49 aufgrund der Erreichbarkeit der Steine in den unteren Kelchgruppen der Niere um bis zu etwa 300° gelenklos abwinkelbar.

Es ist es von Vorteil, den krümmbaren Schaftabschnitt 49 auch seitlich um beispielsweise bis zu 20° oder 25° in zwei entgegengesetzte Richtungen abwinkeln zu können, um den räumlichen Bereich zu vergrößern, in dem insbesondere mit einem Laser der betreffende Stein oder ein anderes Objekt bearbeitet werden kann. Dies wird durch Unterteilung des distalen krümmbaren Schaftabschnitts 49 in einen ersten Bereich 61 und einen zweiten Bereich 63 erreicht, in denen unterschiedlich ausgerichtete Schlitze 55 angeordnet sind. In dem ersten Bereich 61, der sich bis zu dem distalen Ende 51 hin erstreckt, sind die ersten Schlitze 55 jeweils um 180° zueinander versetzt. Dadurch wird, wie vorangehend beschrieben, eine Hauptabwinkelungsebene realisiert. In dem zweiten Bereich 63, der sich proximal vom ersten Bereich 63 erstreckt, sind zweite Schlitze 64 vorgesehen, die in Umfangsrichtung um 90° zu den ersten Schlitzen 55 in dem ersten Bereich versetzt sind. Der zweite Bereich 63 erstreckt sich über eine deutlich kürzere Strecke als der erste Bereich 61, wobei die Abstände der Schlitze 55 und 64 in beiden Bereichen 61 und 63 bevorzugt identisch sind. Die zweiten Schlitze 64 sind axial derart zueinander angeordnet, dass sie abwechselnd auf diametral einander gegenüberliegenden Seiten des Schafts 47 liegen. Folglich ist durch den derart ausgestalteten zweiten Bereich 63 eine eingeschränkte Bewegbarkeit in einer Ebene möglich, die senkrecht zu der Hauptabwinkelungsebene verläuft. Die Bewegbarkeit kann durch die Anzahl der zweiten Schlitze 64 beispielsweise auf einen Winkelbereich von etwa +/- 20° beschränkt werden. Eine Bewegung kann durch zwei weitere mechanische Leitungen 65 und 67 realisiert werden, die direkt distal vor dem zweiten Bereich 63 mit dem Schaft 47 gekoppelt sind und ebenso als Zugdrähte ausgeführt sein könnten. In der Kombination dieser beiden Abwinkelungen wird ermöglicht, dass etwa eine Laserfaser exakt in einem relativ großen Arbeitsbereich das Abscannen eines erkannten Objektes ermöglicht.

In Fig. 2a wird beispielhaft ein leichter Versatz in der Zeichnungsebene nach links erreicht, wie durch den Winkel β angedeutet. Hier wird folglich der erste Bereich 61 und damit die Hauptwinkelebene um etwa 20° zu einer Seite verschwenkt. In Fig. 2b ist indes eine Verschwenkung in die andere Richtung gezeigt, wie durch den Winkel -β angedeutet. Durch den zweiten Bereich 63 mit den darin angeordneten Schlitzen 55 sowie den zusätzlichen mechanischen Leitungen 65 und 67 kann das distale Ende 51 des Schafts 47 deutlich besser bewegt werden. Durch die einstückige Ausführung kann weiterhin gleichzeitig erreicht werden, dass der Schaft 47 eine ausreichende Festigkeit besitzt, um durch Rotieren des Schafts 47 direkt das distale Ende 51 zu bewegen.

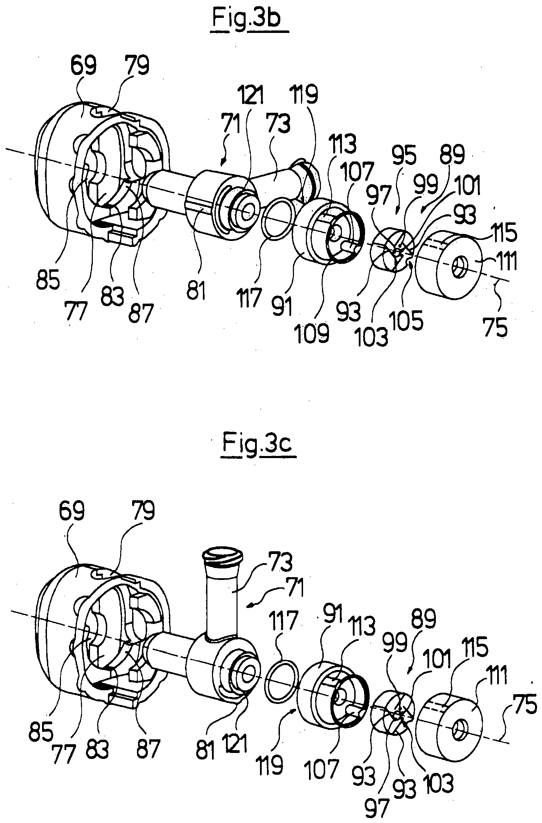

Die Figuren 3a bis 3c zeigen ein Detail einer endoskopischen Vorrichtung, die einen Fluidkanal aufweist, der wie in den vorherigen Figuren als Fluidkanal 7 oder Fluidkanal 53 ausgeführt sein kann. In den Figuren wird allerdings lediglich ein kleiner Teil der Vorrichtung dargestellt, so dass ein Schaft und ein Fluidkanal nicht sichtbar sind. Es wird ein Teil eines Steuergehäuses 69 gezeigt, in das ein Schaftanschlussteil 71 integrierbar ist, mit dem ein Schaft koppelbar ist. Dieser Schaft ist hier nicht dargestellt und kann nach den Prinzipien der Figuren 1, 2a und 2b gestaltet sein. Es kann sich allerdings auch anbieten, einen herkömmlichen Schaft mit dem Schaftanschlussteil 71 zu koppeln, solange er einen vorangehend genannten Fluidkanal aufweist.

An dem Schaftanschlussteil 71 ist eine proximale Fluidkanalöffnung in Form eines Anschlussstutzens 73 angeordnet, der sich an einem äußeren Umfang quer zu einer Schaftanschlussachse 75 nach außen erstreckt. Über den Anschlussstutzen 73 kann Spülflüssigkeit austreten, die aus dem Fluidkanal 7, 53 stammt. Das Schaftanschlussteil 71 ist zur Integration in einen Hohlraum 77 des Steuergehäuses 69 ausgebildet. An den Hohlraum 77 schließt sich eine laterale Öffnung 79 an, durch die sich der Anschlussstutzen 73 nach außen erstreckt. An einer beispielhaft dem Anschlussstutzen 73 gegenüberliegend angeordneten Seite des Schaftanschlussteils 71 ist ein radialer Vorsprung 81 ausgeformt, der in korrespondierend geformte Rastöffnungen 83 und 85 einer Radialkontur 87 des Hohlraums 77 einrasten kann. Die Rastöffnungen 83 und 85 sind beispielhaft um 90° die Schaftanschlussachse 75 umlaufend zueinander versetzt. Das Schaftanschlussteil 71 kann folglich in zwei exemplarisch um 90° voneinander entfernten Drehpositionen einrasten. Die laterale Öffnung 79 kann hierzu korrespondierend ausgeformt sein.

Der Schaft (nicht gezeigt) ist drehfest mit dem Schaftanschlussteil 71 gekoppelt, sodass durch drehen des Schaftanschlussteils 71 auch der Schaft gedreht wird. Dadurch kann die Richtung der Abwinkelung des distalen krümmbaren Schaftabschnitts 9, 49 beeinflusst werden. Da bei Verwendung eines lediglich durch einen Schaftmantel definierten Fluidkanals 7, 53 mehrere Leitungen durch das Steuergehäuse 69 nach außen geführt werden müssen, ohne das Innenlumen des Fluidkanals 7, 53 mit dem Innenlumen des Steuergehäuses 69 zu verbinden, ist ein Dichtmittel in Form einer Dichtungsvorrichtung 89 vorgesehen. Diese ist scheibenartig geformt und ist in eine Dichtungsaufnahme 91 einsetzbar, die wiederum mit dem Schaftanschlussteil 71 fest verbunden wird.

Zum Durchführen von mechanischen Leitungen, beispielsweise Zugdrähten, weist die Dichtungsvorrichtung 89 vier Durchführen in Form von Schlitzen 93 auf. Es werden in diesem Beispiel genau vier Schlitze 93 gezeigt, damit auch ein Schaft nach den Fig. 2a und 2b mit dieser Dichtungsvorrichtung 89 koppelbar ist. Sollte ein anderer Schaft eingesetzt werden, kann selbstverständlich auch eine andere Anzahl von Schlitzen 93 realisiert werden. Die gezeigten Schlitze 93 basieren beispielhaft auf parallelverschobenen Radiallinien, welche um 90° zueinander auf einer von der Dichtungsvorrichtung 89 überdeckten Kreisfläche versetzt sind. Die Schlitze 93 durchsetzen eine Umfangsfläche 95 der Dichtungsvorrichtung 89 und ragen beispielhaft über eine senkrecht zu dem jeweiligen Schlitz 93 verlaufende, einen Mittelpunkt der Dichtungsvorrichtung schneidende Mittellinie hinaus. Sind die Zugdrähte als Flachdrähte ausgeführt, können diese durch jeweils einen Schlitz 93 zwischen dem Schaft und dem Steuergehäuse 69 verlaufen und werden hierbei sanft von dem Material der Dichtungsvorrichtung 89 umschlossen.

Um eine Reibung der Zugdrähte möglichst zu minimieren, ist die Dichtungsvorrichtung 89 bevorzugt aus einem geschlossenzelligen Schaumstoff hergestellt. Die Abmessungen der Dichtungsvorrichtung 89 sind in nicht eingesetztem Zustand bevorzugt etwas größer als die Abmessungen der Dichtungsaufnahme 91, sodass die Dichtungsvorrichtung 89 beim Einsetzen etwas komprimiert werden muss und dann stets in eine entspannte, expandierte Haltung drängt. Hierbei werden sämtliche durchgeführten Elemente dichtend umschlossen.

Die Dichtungsvorrichtung 89 weist ferner weitere Ausnehmungen 97, 99, 101 und 103 auf, die entsprechend der Leitungen 11, 13, 15 und 19 unterschiedliche Abmessungen aufweisen können. Die Dichtungsvorrichtung 89 hat eine größte Ausnehmung 97, die zum ersten Arbeitskanal 15 gehört, der sich entlang der Erstreckungsachse 75 durch das Steuergehäuse 69 zur ersten proximalen Arbeitskanalöffnung 39 erstrecken kann. Benachbart zur größten Ausnehmung 97 sind drei weitere Durchführungen 99, 101 und 103 in Form von Ausnehmungen angeordnet, welche der Durchführung der Leitungen 11, 13 und des kleineren zweiten Arbeitskanals 19 dient. Die Durchführung 103, die exemplarisch die kleinsten Abmessungen aufweist, ist etwa zum Durchführen des kleineren zweiten Arbeitskanals 19 mit einer Abmessung von deutlich unterhalb 1 mm, beispielsweise 0,55 mm oder weniger, geeignet. Die beiden anderen Durchführungen 99 und 101 gehören zu den elektrischen Leitungen 11, 13, die mit der distalseitigen LED bzw. mit dem Bildsensor verbunden sind. Die Abmessungen der Ausnehmungen 97, 99, 101 und 103 sind jeweils etwas kleiner als der Querschnitt der zugehörigen durchgeführten Leitung 11, 13, 15 und 19 ausgebildet, um einen Dichtungseffekt außenseitig an der Leitung 11, 13, 15 und 19 zu erzielen. Die Dichtungsvorrichtung 89 ist vorzugsweise als elastischer Zellschaumblock aus Ethylen-Propylen-Dien-Kautschuk (EPDM) ausgebildet, sodass sich die Ausnehmungen 97, 99, 101 und 103 entsprechend bei Durchführung der Leitungen 11, 13, 15 und 19 aufdehnen und diese jeweils dichtend umschließen.

Die Dichtungsvorrichtung 89 weist eine radiale Vertiefung 105 auf, die korrespondierend zu einem radialen Vorsprung 107 in einem hülsenartigen Abschnitt 109 der Dichtungsaufnahme 91 ausgebildet ist. Hierdurch wird stets die rotatorische Position der Dichtungsvorrichtung 89 fixiert, sodass beim Verdrehen des Schaftanschlussteils 71 die Dichtungsvorrichtung 89 der Drehung folgt.

Es ist sinnvoll, in der Dichtungsaufnahme 91 und einem daran zu befestigenden Deckel 111 Führungsmittel zum Führen der Zugdrähte anzuordnen, sodass die Schlitze 93 der Dichtungsvorrichtung 89 beim Verdrehen des Schaftanschlussteils 71 stets mit den Zugdrähten fluchten. Die Dichtungsvorrichtung 89 wird daher ausschließlich durch eine Axialkraft der Zugdrähte belastet. Um weiterhin eine ideale Führung der Zugdrähte zu erreichen, ist der Deckel 111 verdrehfest an der Dichtungsaufnahme 91 angeordnet. Dazu kann an dem hülsenartigen Abschnitt 109 eine radial außenliegende Feder 113 aufweisen, die mit einer radial innenliegenden Vertiefung 115 des Deckels 111 fluchtet.

Wie in Fig. 3b dargestellt, kann ein zusätzlicher Dichtring 117 vorgesehen sein, der die Dichtungsaufnahme 91 an dem Schaftanschlussteil 71 abdichtet. Dazu weist die Dichtungsaufnahme 91 an einer dem hülsenartigen Abschnitt 109 entgegengesetzt angeordneten Vertiefung 119 eine Dichtfläche auf, die mit dem Dichtungsring 117 und einer ringförmigen Fläche 121 des Schaftanschlussteils 71 in Anschlag bringbar ist. Die Spülflüssigkeit, die über den Spülkanal proximalwärts in das Schaftanschlussteil 71 fließt, kann die Dichtungsvorrichtung 89 nicht proximalwärts passieren und fließt über den Anschlussstutzen 73 radialwärts ab.

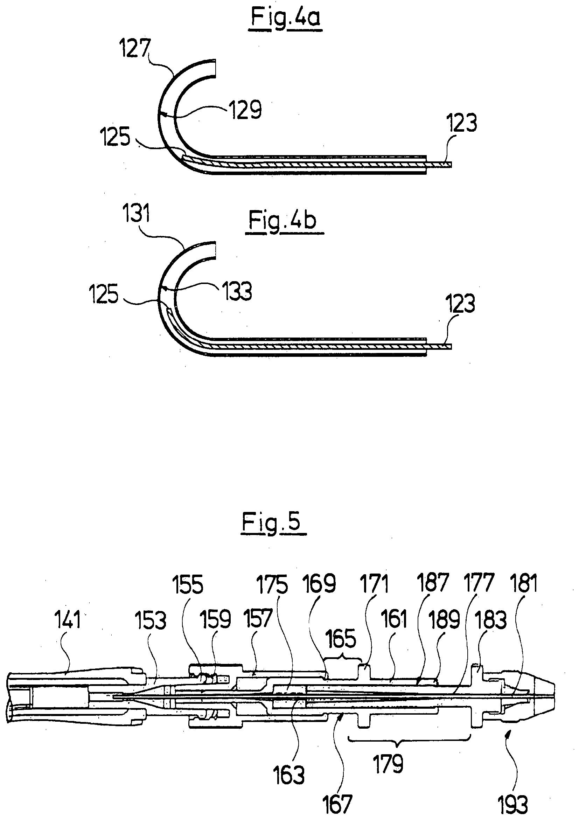

In Fig. 4a und 4b wird ein weiterer Aspekt des endoskopischen Instruments gezeigt. In Figur 4a ist ein Lichtleiter 123 dargestellt, der insbesondere ein Laserlichtleiter in Form einer Einzelfaser ist und einen exemplarischen Durchmesser von 0,45 mm aufweist. Dieser Durchmesser kann vorzugsweise eine Schutzschicht des Lichtleiters 123 beinhalten, wobei der lichtleitende Kerndurchmesser beispielsweise nur 0,272 mm betragen kann. Selbstverständlich kann der Lichtleiter 123 auch einen noch geringeren oder einen etwas größeren Durchmesser besitzen. Der Lichtleiter 123 weist ein Lichtaustrittsende 125 auf, welches für optimale Abstrahlungseigenschaften möglichst scharfkantig und senkrecht zu der Erstreckungsachse des Lichtleiters 123 verläuft. Es ist allerdings mit Schwierigkeiten verbunden, einen solchen Lichtleiter 123 durch einen Arbeitskanal 127 zu schieben, der etwa einen Durchmesser von 1,2 mm oder mehr aufweist. Das scharfkantige Lichtaustrittsende 125 könnte sich beim Durchschieben durch den Arbeitskanal 127 mit dessen Innenwandung 129 verkanten und das Durchschieben verhindern oder der Lichtleiter 123 könnte sogar knicken. Ferner könnte die Wandung 129 beim Verkanten auch zerstört werden, wenn sich das Lichtaustrittsende 125 in die Innenwandung 129 bohrt.

Um dies zu verhindern, wird, wie in Fig. 4b dargestellt, ein alternativer Arbeitskanal 131 als zweiter, kleinerer Arbeitskanal 19 für den Lichtleiter 123 bereitgestellt, der zumindest in einem abwinkelbaren Abschnitt einen Innendurchmesser von nur wenig mehr als dem Durchmesser des Lichtleiters 123 (hier 0,45mm) aufweist und beispielhaft 0,55 mm beträgt. Der Lichtleiter 123 erfährt an seinem Lichtaustrittsende 125 lediglich einen sehr geringen Angriffswinkel zu einer Innenwandung 133 des Arbeitskanals 133, sodass ein Verkanten zuverlässig verhindert wird.

Die genannten Durchmesser im abwinkelbaren Abschnitt sind lediglich beispielhaft zu verstehen. Der Innendurchmesser des Arbeitskanals 131 sollte zum Durchführen des Lichtleiters 123 zumindest im abwinkelbaren Abschnitt einen Innendurchmesser aufweisen, der den Außendurchmesser des Lichtleiters 123 nicht allzu sehr überschreitet. Der Unterschied zwischen den beiden Durchmessern sollte im abwinkelbaren Abschnitt nicht zu groß sein, da sonst die Gefahr des Verkantens und/oder des Beschädigens des Arbeitskanals 131 besteht. Es hat sich herausgestellt, dass ein Durchmesserunterschied im abwinkelbaren Abschnitt von maximal 30 % das Beschädigen des Arbeitskanals 131 verhindern kann. Der Innendurchmesser des Arbeitskanals 131 sollte daher zumindest im abwinkelbaren Abschnitt den Außendurchmesser des Lichtleiters 123 um höchstens 30% überschreiten, was mit den genannten beispielhaften Durchmessern der Fall ist.

In den Figuren 5 bis 6c wird ein weiterer Aspekt zur Realisierung an einem endoskopischen Instrument 134 gezeigt. Fig. 6a bis 6c zeigen eine Handhabungseinrichtung 135, an die ein proximales Ende 138 eines Schafts 137 angeschlossen ist. Dieser weist ein distales Ende 139 auf, welches in der Zeichnungsebene links etwas vergrößert dargestellt ist und beispielhaft eine erste distale Arbeitskanalöffnung 140 aufweist. An einer in der Zeichnungsebene unteren Seite ist eine proximale Fluidkanalöffnung 142 sowie ein elektrisches Anschlusskabel 146 angeordnet, wobei aus der proximalen Fluidkanalöffnung 142 eine Spülflüssigkeit oder dergleichen austreten kann. Hier ist an der oberen Seite ein separater Fluideinlass 148 angeordnet, durch den der erste Arbeitskanal 15 mit Spülflüssigkeit durchspült werden kann. Selbstverständlich kann der Schaft 137 ebenso einen distalen krümmbaren Schaftabschnitt aufweisen, der wie in den vorhergehenden Figuren dargestellt mit Schlitzen versehen ist. Generell ist es von Vorteil, den Schaft 3 zum distalen Ende 139 hin gelenklos abwinkelbar auszugestalten.

An einem proximalen Ende der Handhabungseinrichtung 135 sind ein erster Schenkel 141 und ein zweiter Schenkel 143 in einer Y-Anordnung vorgesehen, zwischen denen eine Anlagefläche 145 zum Anlegen eines Daumenballens einer Hand vorgesehen ist. An dem zweiten Schenkel 143 ist beispielhaft eine zweite proximale Arbeitskanalöffnung 144 vorgesehen. Die Ausrichtung der beiden Schenkel 141 und 143 zueinander sowie die Dimensionierung der Anlagefläche 145 sind derart gewählt, dass beim Greifen der Handhabungsvorrichtung 135 der Daumenballen derart an der Anlagefläche 145 anliegt, dass die Handhabungseinrichtung 135 eine direkte Verlängerung eines Unterarms des Benutzers bildet. Der Benutzer kann folglich die Handhabungsvorrichtung 135 sehr bequem ausschließlich mittels Supination und Pronation des Unterarms um die Längsachse des Schafts 137 verdrehen, ohne eine komplexere Armbewegung ausführen zu müssen. Insbesondere bei herkömmlichen pistolenartigen Handhabungsvorrichtungen ist dies nämlich nicht der Fall.

Distalwärts folgen an zwei einander entgegengesetzten Seiten der Handhabungseinrichtung 135 ein oberer Abzug 147 und ein unterer Abzug 149, die von zwei Fingern der Hand durch proximalwärtiges Ziehen betätigt werden können und jeweils mit einem distalwärts verlaufenden Zugdraht verbinden sind. Wie in dem Ausführungsbeispiel in Fig. 1 könnten auch der obere Abzug 147 und der untere Abzug 149 miteinander gekoppelt sein, so dass beide Abzüge 147 und 149 gegenläufige Bewegungen ausführen. Es ist vorstellbar, dass zwischen den Abzügen 147 und 149 eine Führungsrolle vorliegt, um die genannten Zugdrähte jeweils als ein Abschnitt eines gemeinsamen Zugdrahts ausgeführt sind, der um die Führungsrolle herumgeführt ist. Allerdings wäre auch eine Steuerscheibe denkbar, die mit beiden Abzügen 147 und 149 sowie jeweils einem separaten Zugdraht gekoppelt ist. Zum Arretieren einer momentanen Position der Führungsrolle bzw. der einzelnen Zugdrähte könnte eine Arretiereinrichtung 151 vorgesehen sein. In dem dargestellten Fall ist die Arretiereinrichtung 151 als Rändelschraube ausgeführt, die beispielsweise in eine Steuerscheibe ragt und durch Festziehen an die äußere Oberfläche der Handhabungseinrichtung 135 geklemmt wird.

Beispielhaft befindet sich an dem ersten Schenkel 141 gemäß einem weiteren Aspekt dieser Offenbarung als Teil eines Luer-Lock Anschlusses ein Arbeitskanaleinlass 153, an dem proximal ein erstes Verschlussgewinde 155 angeordnet ist. Durch den Arbeitskanaleinlass 153 ist ein Schaftwerkzeug in Form eines Fangkorbeinsatzes in das Instrument 1 eingeschoben und angeschlossen. Der Fangkorbeinsatz weist ein Anschlusselement 157 auf, das über ein zweites Verschlussgewinde 159, das mit dem ersten Verschlussgewinde 155 korrespondierend ausgeformt ist, verbunden ist. Am Anschlusselement 157 des Fangkorbeinsatzes ist ein erstes drehbares Aufnahmeteil 161 gelagert, das mit einem Schlauch 163 als Mantelelement des Fangkorbeinsatzes verbunden ist. Das Mantelelement 163 des Fangkorbeinsatzes erstreckt sich durch den ersten Arbeitskanal 15 durch den Schaft 3. Das erste Aufnahmeteil 161 weist einen ersten Verschiebeabschnitt 165 auf, der mit einer umfangseitigen Rasterung 167 versehen ist. Diese erlaubt das Einrasten in unterschiedlichen Positionen in einer Öffnungskontur 169 des Anschlusselements 157. Durch Verschieben des ersten Aufnahmeteils 161 in distalwärtiger Richtung entlang des ersten Verschiebeabschnitts 165 kann folglich der Schlauch 163 im ersten Arbeitskanal 15 distalwärts verschoben werden. An einem von dem Anschlusselement 157 entgegengesetzten Ende des ersten Verschiebeabschnitts 165 ist ein erster Absatz 171 in Form eines umlaufenden Kragens angeordnet, der eine umfangsseitige Rändelung 173 aufweist. Hierdurch kann das erste Aufnahmeteil 161 gegriffen und gedreht werden. Die umfangsseitige Rasterung 167 ist daher bevorzugt in Form von parallel zueinander angeordneten Rillen ausgeführt, die einen abgerundeten Profilquerschnitt aufweisen. Ein manuelles Verschieben in distalwärtiger oder proximalwärtiger Richtung kann jeweils zum Lösen und Erreichen eines Einrastens führen.

In einem Innenlumen 175 des ersten Aufnahmeteils 161 ist ein zweites Aufnahmeteil 177 angeordnet. Dieses weist einen zweiten Verschiebebereich 179 auf, mit dem die Relativposition des zweiten Aufnahmeteils 177 zu dem ersten Aufnahmeteil 161 einstellbar ist. Gleichzeitig ist das zweite Aufnahmeteil 177 über eine drehfeste, verschiebbare Verbindung mit dem ersten Aufnahmeteil 161 verbunden. Das zweite Aufnahmeteil 177 folgt daher unmittelbar der Bewegung des ersten Aufnahmeteils 161. Das zweite Aufnahmeteil 177 ist mit einem Zugdraht 181 verbunden, der sich durch das als Schlauch 163 ausgeführte Mantelelement des Fangkorbeinsatzes erstreckt. Das zweite Aufnahmeteil 177 weist weiterhin an einem von dem ersten Aufnahmeteil 161 abgewandten Ende des zweiten Verschiebeabschnitts 179 einen zweiten Absatz 183 in Form eines umlaufenden Kragens auf, der ebenso mit einer Rändelung 185 versehen ist. Ein Benutzer kann folglich die Aufnahmeteile 161 und 177 gut greifen und verdrehen. Aufgrund der festen Verbindung zwischen den Komponenten werden auch der Schlauch 163 und der Zugdraht 181 gedreht. Das zweite Aufnahmeteil 177 weist ebenso eine Rasterung 187 auf, die mit einer Öffnungskontur 189 des ersten Aufnahmeteils 161 verrasten kann. Die Position des zweiten Aufnahmeteils 177 kann folglich stets fest zu dem ersten Aufnahmeteil 161 eingestellt werden.

Wie in den Figuren 6a bis 6c dargestellt, kann durch diese Ausgestaltung eine besonders vorteilhafte Konstruktion eines Fangkorbs 191 realisiert werden, der in dem Mantelelement 163 des Fangkorbeinsatzes angeordnet und von diesem geschützt ist. Der Fangkorb 191 ist so elastisch ausgebildet, dass er vollständig in das Mantelelement 163 einziehbar ist und durch distalwärtige Bewegung aus diesem herausschiebbar ist, wobei er sich zu seiner vollen Größe ausdehnt. Durch gezieltes Bewegen des Fangkorbs 191 kann ein Nierenstein oder ähnliches gegriffen werden, wobei das Greifen durch proximalwärtiges Ziehen des Zugdrahts 181 erfolgt und der Fangkorb sich dadurch wieder komprimiert. Fig. 6a zeigt das erste Aufnahmeteil 161 und das zweite Aufnahmeteil 177 in proximaler Position eingerastet. Dies bedeutet, dass ein Betätigungsgriff 193 so weit wie möglich aus der Handhabungseinrichtung 135 herausgezogen ist.

Wie in der vergrößerten Darstellung der distalen Spitze 139 des Schafts 137 ersichtlich, ragt das Mantelelement 163 des Fangkorbeinsatzes nicht über die distale Schaftspitze 139 hinaus. Durch Bewegen des ersten Aufnahmeteils 161 über den vollständigen ersten Verschiebeweg 165 in distalwärtiger Richtung, sodass der erste Absatz 171 auf der Anschlusseinrichtung 157 bündig aufliegt, ragt der Schlauch 163 beispielsweise um etwa 5 mm aus der distalen Schaftspitze 139 heraus. Durch Einschieben des zweiten Aufnahmeteils 177 in distalwärtiger Richtung kann der Fangkorb 191 aus dem Schlauch 163 herausgeschoben werden, so dass er sich entfaltet. Als Verschiebeweg für den Schlauch 163 könnten sich 5 mm eignen. Es ist denkbar, dass sich zum Entfalten des Fangkorbs 191 eine deutlich größere Länge anbietet, beispielsweise 20 mm.

Der besondere Vorteil dieser Anordnung mit dem distalwärts verschiebbaren Mantelelement 163 des Fangkorbeinsatzes und der drehfesten Verbindung liegt darin, dass der Fangkorb 191 in sämtlichen Entfaltungszuständen ebenso sehr leicht radial durch Einwirken auf den Handgriff 193 gedreht werden kann. Gleichzeitig wird durch das Betätigen des Handgriffs 193 auch ein Vorschieben und Entfalten des Fangkorbs 191 durch die Hand erlaubt, die gerade nicht die Handhabungseinrichtung 135 greift. Ist ein Objekt durch den Drahtfangkorb 191 erfasst, kann durch Zurückziehen des zweiten Aufnahmeteils 177 das Objekt festgehalten werden. Aufgrund der Rasterung 187 bleibt das Objekt gefangen, ohne dass es aktiv festgehalten werden muss. Der Fangkorb kann demnach sehr leicht von einem einzelnen Benutzer betätigt werden und ein zweiter Benutzer, mit dem während der Operation intensiv kommuniziert werden muss, ist nicht erforderlich.

Durch den zweiten, kleineren Arbeitskanal 19 kann parallel zum Fangkorbeinsatz im ersten Arbeitskanal 15 ein Laserlichtleiter 123 an die distale Schaftspitze 17 geführt werden, um beispielsweise einen Nierenstein mittels Laserlicht zertrümmern zu können. Der zweite, kleinere Arbeitskanal 19 endet proximalseitig an der zweiten proximalen Arbeitskanalöffnung 144 am zweiten Schenkel 143 der Handhabungseinrichtung 135.

In den Figuren 7a-f ist der Schaft 3, insbesondere der krümmbare Schaftabschnitt 9, 49 des Schafts 3 und das distale Schaftende 17, 51, 139 genauer gezeigt. In Figuren 7a-c ist eine distale Endhülse 195 nicht gezeigt, die in Figuren 7d-f genauer gezeigt ist. Das distale Schaftende 17, 51, 139 weist eine Anschlusshülse 197 auf, mittels derer die Endhülse 195 am Schaft 3 drehfest formschlüssig befestigt ist (siehe Fig. 7f). Die Endhülse 195 weist eine Ausnehmung 199 und die Anschlusshülse 197 eine zur Ausnehmung 199 korrespondierend passend geformte Ausbuchtung 201 auf, wodurch eine drehfeste formschlüssige Verbindung zwischen Endhülse 195 und Anschlusshülse 197 erreicht wird.

Die Anschlusshülse 197 überlappt in einem Überlappungsabschnitt das distale Ende des geschlitzten krümmbaren Schaftabschnitts 9, 49. In dem Überlappungsabschnitt sind laterale Spülöffnungen 203 vorhanden, hierin Form von acht umlaufend verteilten radialen Durchbohrungen. Da die Schlitze 33, 55, 64 des krümmbaren Schaftabschnitts 9, 49 hier von einem Schutzschlauch 205 umgeben sind (siehe Fig. 7f, nicht gezeigt in Fig. 7a-e), dienen die Spülöffnungen 203 als distaler Spülauslass oder Spüleinlass des Fluidkanals 7, 53. Die Dicke der Anschlusshülse 197 entspricht in etwa der Dicke des Schutzschlauchs 205, sodass die radialen Außenflächen von Schutzschlauch 205, Anschlusshülse 197 und Endhülse 195 an Stoß gesetzt miteinander kantenlos fluchten (siehe Fig. 7f).

In Fig. 7b sind auch die Zugdrähte 57, 59, 65, 67 gezeigt, die sich diametral gegenüberliegend (hier: oben 57, 65 und unten 59, 67) an der Schaftinnenfläche durch den Schaft 3 erstrecken, um den krümmbaren Schaftabschnitt 9, 49 gelenklos nach oben oder unten abwinkeln zu können. Die Schlitze 33, 55, 64 sind dazu keilförmig ausgestaltet, sodass die lichte Weite der Schlitze 33, 55, 64 mittig, wo die Zugdrähte 57, 59, 65, 67 verlaufen, am größten ist und sich zu den Enden hin in Umlaufrichtung verjüngt. Die Enden der Schlitze 33, 55, 64 weisen Rundausnehmungen auf, um bei Verbiegen des Schaftmaterials die Spannungen zu verringern und Materialrisse bzw. das Risiko plastischer Verformungen des Schaftmaterials zu verringern. Die gelenklose Abwinklung des Schaftabschnitts 9, 49 soll durch möglichst elastische Verformung des geschlitzten Schaftmaterials erfolgen, das eine Rückstellwirkung in die gerade Schaftform hat.

In Fig. 7d ist die Anordnung von einer Lichtquelle in Form einer LED 207, eines Objektivs 209 mit dahinterliegendem Bildsensor, der ersten distalen Arbeitskanalöffnung 18, 52, 140 des ersten Arbeitskanals 15 und einer zweiten distalen Arbeitskanalöffnung 211 des zweiten Arbeitskanals 19 in der distalen Endhülse 195 in einer Frontansicht gezeigt. Es sei hier auf die extrem kleinen Größenverhältnisse hingewiesen. Der Außendurchmesser der distalen Endhülse 195 kann 3 mm oder weniger betragen. Die LED 207 kann 0,55 mm breit und der Bildsensor 209 unter 1 mm breit sein. Die erste distale Arbeitskanalöffnung 18, 52, 140 kann einen Innendurchmesser von 1,2 mm oder weniger und die zweite distale Arbeitskanalöffnung 211 einen Innendurchmesser von 0,55 mm oder weniger aufweisen. An der zweiten distalen Arbeitskanalöffnung 211 kann die distalseitige Lichtauskopplung eines durch den zweiten Arbeitskanal 19 durchgeschobenen Laserlichtleiters 123 platziert werden. Aus der ersten distalen Arbeitskanalöffnung 18, 52, 140 kann der Werkzeugkopf eines durch den ersten Arbeitskanal 15 durchschiebbaren Schaftwerkzeugs, beispielsweise ein Fangkorbeinsatz oder ein Zangen- oder Schereninstrument, geführt werden. Der erste Arbeitskanal 15 kann außerdem als Zu- oder Abführleitung von Spülflüssigkeit verwendet werden, die über die lateralen Spülöffnungen 203 und den Fluidkanal 7, 53 entsprechend abgeführt bzw. zugeführt wird. Bevorzugt ist eine Zufuhr von klarer Spülflüssigkeit über den ersten Arbeitskanal 15 und eine Abfuhr über die lateralen Spülöffnungen 203 und den Fluidkanal 7, 53, um eine klare distalwärtige Sicht zu haben.

In Fig. 7e ist gezeigt, dass die distale Endhülse 195 frontseitig abgeschrägt ist, sodass insbesondere die erste distale Arbeitskanalöffnung 18, 52, 140 unter einem Winkel zur Längsachse des Schafts 3 verläuft. Vorzugsweise ist auch die zweite distale Arbeitskanalöffnung 211 abgeschrägt (nicht sichtbar in Fig. 7e). Auch die entsprechenden distalen Öffnungen in der Endhülse 195 für die LED 207 und das Objektiv 209 können abgeschrägt sein, um die jeweilige Ausleuchtung bzw. den Sichtwinkel zu verbessern. Die eine oder mehr Abschrägungen der distalen Schaftspitze 17, 51, 139 haben den weiteren Vorteil, dass die distale Schaftspitze 17, 51, 139 einfacher durch einen Harn- oder Nierenleiter bzw. einen Trokar oder Katheter geführt werden kann.

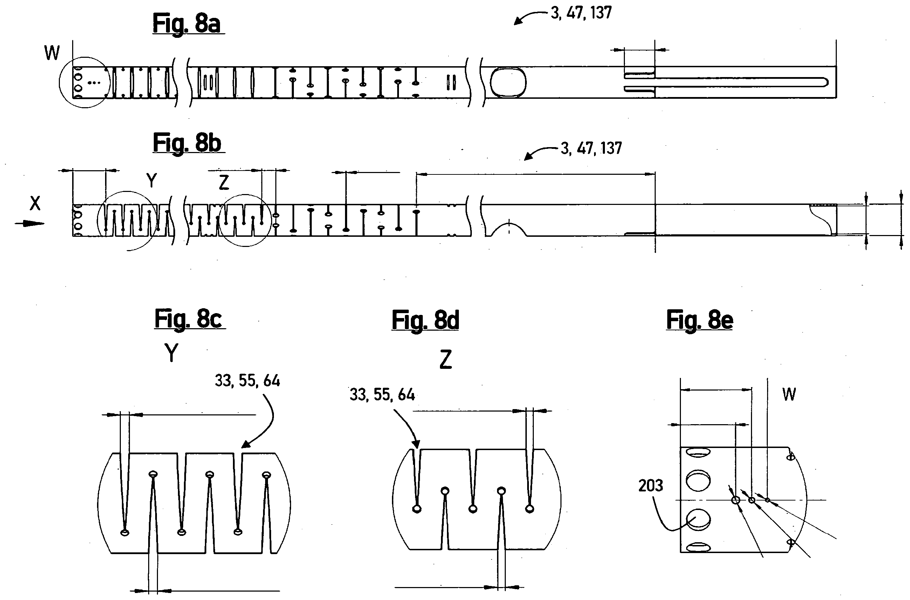

Figuren 8a-e zeigen den einteiligen metallischen rohrförmigen Hauptkörper des Schafts 3, 47, der die Spülöffnungen 203, die Schlitze 33, 55, 64 und die proximale Fluidkanalöffnung 45, 73, 142 auf. Der krümmbare Schaftabschnitt 9, 49 mit den Schlitzen 33, 55, 64 weist erfindungsgemäß mindestens zwei Unterabschnitte Y, Z auf, wobei die axialen Abstände zwischen den Schlitzen 33, 55, 64 in den Unterabschnitten X, Z unterschiedlich sind. Die Abstände zwischen den Schlitzen 33, 55, 64 sind dabei in einem ersten Unterabschnitt Y kleiner als in einem zweiten Unterabschnitt Z, wobei der erste Unterabschnitt Y distal vom zweiten Unterabschnitt Z angeordnet ist. Dadurch kann im ersten Unterabschnitt Y eine stärkere Krümmung erzielt werden als im zweiten Unterabschnitt Z. Außerdem ist der erste Unterabschnitt Y weniger biegesteif als der Unterabschnitt Z, sodass sich der krümmbare Schaftabschnitt 9, 49 bei der Krümmung von der distalen Spitze 17, 51, 139 aus einrollt. Dadurch wird eine besonders gute Beweglichkeit der distalen Spitze 17, 51, 139 und ein besonders großer Abwinkelbereich von bis zu 300° auf kleinem Raum erreicht. Wie in Fig. 8c,d gezeigt, ist zudem erfindungsgemäß die lichte Weite der Schlitze 33, 55, 64 im ersten Unterabschnitt Y größer als im zweiten Unterabschnitt Z. Dies unterstützt ebenfalls die sich in distalwärtige Richtung verbessernde Krümmbarkeit des Schaftabschnitts 9, 49. Die lichte Weite und/oder die Schlitzabstände können innerhalb und/oder zwischen den Unterabschnitten Y, Z graduell variieren, sodass die Unterabschnitten Y, Z ineinander verlaufen können. Die Unterabschnitte Y, Z, können benachbart zueinander oder getrennt voneinander angeordnet sein.

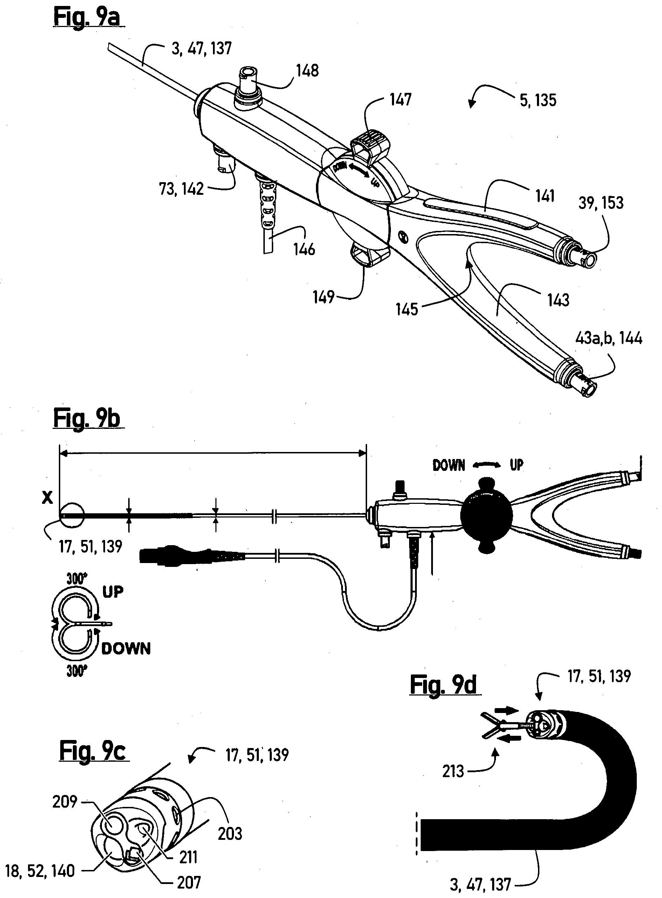

Figuren 9a,b zeigen ein weiteres Design der Y-förmigen Handhabungseinrichtung 5, 135. Das in Fig. 9b gezeigte Instrument kann als werkseitig vormontiertes und sterilisiertes Einwegprodukt günstig hergestellt werden. Es ist noch einmal verdeutlicht, wie durch Ziehen der Abzüge 147, 149 die distale Schaftspitze 17, 51, 139 um bis zu 300° nach oben bzw. nach unten gekrümmt werden kann. Fig. 9c zeigt die distale Schaftspitze 17, 51, 139 in perspektivischer Ansicht mit abgeschrägten Arbeitskanalöffnungen 18, 52, 140, 144. Fig.9d verdeutlicht, wie ein Schaftwerkzeug in beispielhafter Form eines Zangeninstruments 213 durch den ersten Arbeitskanal 15 durchgeschoben werden kann und bei gekrümmtem Schaftabschnitt 9, 49 axial positionierbar ist.

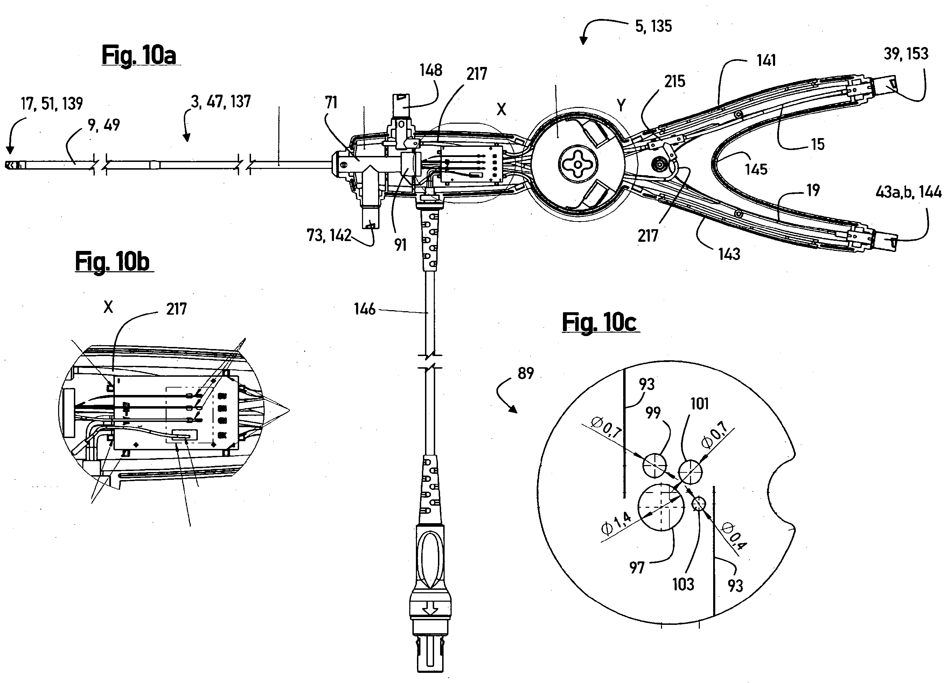

In Figuren 10a,b ist das Innenleben der Y-förmigen Handhabungseinrichtung 5, 135 genauer im Längsschnitt gezeigt. Fig. 10b zeigt dabei einen vergrößerten Ausschnitt X. Fig. 10c zeigt eine vergrößerte Darstellung der Dichtungsvorrichtung 89 in Form eines Zellschaumblocks, der in der Dichtungsaufnahme 91 sitzt und den Fluidkanal 7, 53 proximalseitig abdichtet. Die Dichtungsvorrichtung 89 weist Ausnehmungen 97, 99, 101 und 103 zur Durchführung der Leitungen 11, 13, 15 und 19 auf, die entsprechend den Leitungen 11, 13, 15 und 19 unterschiedliche Abmessungen aufweisen. Die Dichtungsvorrichtung 89 hat eine größte Ausnehmung 97, die zum ersten Arbeitskanal 15 gehört, der sich entlang der Erstreckungsachse 75 durch das Steuergehäuse 69 zur proximalen Arbeitskanalöffnung 39 erstrecken kann. Benachbart zur größten Ausnehmung 97 sind drei weitere Durchführungen 99, 101 und 103 in Form von Ausnehmungen angeordnet, welche der Durchführung der Leitungen 11, 13 und des kleineren zweiten Arbeitskanals 19 dient. Die Durchführung 103, die exemplarisch die kleinsten Abmessungen aufweist, ist etwa zum Durchführen des kleineren zweiten Arbeitskanals 19 mit einer Abmessung von deutlich unterhalb 1 mm, beispielsweise 0,55 mm oder weniger, geeignet. Die beiden anderen Durchführungen 99 und 101 gehören zu den elektrischen Leitungen 11, 13, die mit der distalseitigen LED 207 bzw. mit dem Bildsensor 209 verbunden sind. Die Abmessungen der Ausnehmungen 97, 99, 101 und 103 sind jeweils etwas kleiner als der Querschnitt der zugehörigen durchgeführten Leitung 11, 13, 15 und 19 ausgebildet, um einen Dichtungseffekt außenseitig an der Leitung 11, 13, 15 und 19 zu erzielen. Die Dichtungsvorrichtung 89 ist vorzugsweise als elastischer Zellschaumblock aus Ethylen-Propylen-Dien-Kautschuk (EPDM) ausgebildet, sodass sich die Ausnehmungen 97, 99, 101 und 103 entsprechend bei Durchführung der Leitungen 11, 13, 15 und 19 aufdehnen und diese jeweils dichtend umschließen. Die zwei zueinander versetzt angeordneten Schlitze 93 dienen der dichten Durchführung der zwei Seilzüge 57, 59. Die radiale Vertiefung 105 dient der Drehsicherung der Dichtungsvorrichtung 89.

In Fig. 10a ist zudem verdeutlicht, wie der erste Arbeitskanal 15 über den oberseitigen Fluideinlass 148 mit Spülflüssigkeit versorgt werden kann, sodass der erste Arbeitskanal 15 als Zuführkanal von Spülflüssigkeit dienen kann. Dazu ist innerhalb der Handhabungseinrichtung 5, 135 ein T-Anschlussstück 215 im ersten Arbeitskanal 15 angeordnet, das über eine Spülleitung 217 mit dem oberseitigen Fluideinlass 148 verbunden ist. Somit kann ein hochgehängter Tropf oder eine Pumpe an den Fluideinlass 148 angeschlossen werden, sodass Spülflüssigkeit durch die Spülleitung 217 in den ersten Arbeitskanal 15 läuft.

Es sei angemerkt, dass die Merkmale der zuvor beschriebenen Ausführungsbeispiele miteinander beliebig kombinierbar sind.

### Bezugszeichenliste:

- 1: Endoskopisches Instrument
- 3: Schaft
- 4: proximales Ende des Schafts
- 5: Handhabungseinrichtung
- 7: Fluidkanal
- 9: krümmbarer Schaftabschnitt
- 11: erste optische oder elektrische Leitung
- 13: zweite optische oder elektrische Leitung
- 15: erster Arbeitskanal
- 17: distales Ende / distale Spitze des Schafts
- 18: erste distale Arbeitskanalöffnung
- 19: zweiter Arbeitskanal
- 21: Strömungslinien / Rückströmung Fluid
- 23: Gehäuse
- 25: Grifföffnung
- 27: Auflagefläche
- 29: unterer Abzug / Bedienhebel
- 31: oberer Abzug / Bedienhebel
- 33: Schlitz
- 35: Umfangsfläche
- 37: obere Seite der Handhabungseinrichtung
- 39: proximales Ende
- 41: untere Seite der Handhabungseinrichtung
- 43a, 43b: zweite proximale Arbeitskanalöffnung
- 45: proximale Fluidkanalöffnung
- 47: Schaft
- 49: krümmbarer Schaftabschnitt
- 51: distales Ende des Schafts
- 52: erste distale Arbeitskanalöffnung
- 53: Fluidkanal
- 55: erster Schlitz
- 57: mechanische Leitung (Zugdraht)
- 59: mechanische Leitung (Zugdraht)
- 61: erster Bereich
- 63: zweiter Bereich
- 64: zweiter Schlitz
- 65: mechanische Leitung (Zugdraht)
- 67: mechanische Leitung (Zugdraht)
- 69: Steuergehäuse
- 71: Schaftanschlussteil
- 73: proximale Fluidkanalöffnung / Anschlussstutzen
- 75: Schaftanschlussachse
- 77: Hohlraum
- 79: laterale Öffnung
- 81: radialer Vorsprung
- 83: Rastöffnung
- 85: Rastöffnung
- 87: Radialkontur
- 89: Dichtungsvorrichtung
- 91: Dichtungsaufnahme
- 93: Schlitz / Durchführung
- 95: Umfangsfläche
- 97: Durchführung
- 99: Durchführung
- 101: Durchführung
- 103: Durchführung
- 105: radiale Vertiefung
- 107: radialer Vorsprung
- 109: hülsenartiger Abschnitt
- 111: Deckel
- 113: radial außenliegende Feder
- 115: radial innenliegende Vertiefung
- 117: Dichtungsring
- 119: Vertiefung
- 121: ringförmige Fläche
- 123: Lichtleiter
- 125: Lichtaustrittsende
- 127: herkömmlicher Arbeitskanal
- 129: Innenwandung
- 131: Arbeitskanal
- 133: Innenwandung
- 134: endoskopisches Instrument
- 135: Handhabungseinrichtung
- 137: Schaft
- 138: proximales Ende des Schafts
- 139: distales Ende des Schafts
- 140: erste distale Arbeitskanalöffnung
- 141: erster Schenkel
- 142: proximale Fluidkanalöffnung
- 143: zweiter Schenkel
- 144: proximale zweite Arbeitskanalöffnung
- 145: Anlagefläche
- 146: elektrisches Anschlusskabel
- 147: oberer Abzug / Bedienhebel
- 148: Fluideinlass
- 149: unterer Abzug / Bedienhebel
- 151: Arretiereinrichtung
- 153: Arbeitskanaleinlass
- 155: erstes Verschlussgewinde
- 157: Anschlusselement
- 159: zweites Verschlussgewinde
- 161: erstes Aufnahmeteil
- 163: Mantelelement des Schaftwerkzeugs
- 165: erster Verschiebeabschnitt
- 167: Rasterung
- 169: Öffnungskontur
- 171: erster Absatz / Kragen
- 173: Rändelung
- 175: Innenlumen
- 177: zweites Aufnahmeteil
- 179: zweiter Verschiebebereich
- 181: Zugdraht
- 183: zweiter Absatz / Kragen
- 185: Rändelung
- 187: Rasterung
- 189: Öffnungskontur
- 191: Fangkorb
- 193: Betätigungsgriff
- 195: Endhülse
- 197: Anschlusshülse
- 199: Ausnehmung
- 201: Ausbuchtung
- 203: Spülöffnungen
- 205: Schutzschlauch
- 207: LED
- 209: Objektiv
- 211: zweite distale Arbeitskanalöffnung
- 213: Zangeninstrument
- 215: T-Anschlussstück
- 217: Fluidleitung

- β: Winkel (seitlicher Versatz)

## Patentansprüche

1. Endoskopisches Instrument (1,134) zum Einführen in einen Körper eines Patienten, wobei das Instrument (1,134) einen rohrförmigen Schaft (3, 47, 137) und mindestens zwei durch den Schaft (3, 47, 137) verlaufende elektrische, mechanische und/oder optische Leitungen (11, 13, 15, 19, 57, 59, 65, 67, 123, 181) aufweist, wobei die Leitungen (11, 13, 15, 19, 57, 59, 65, 67, 123, 181) mindestens zwei Zugdrähte (57, 59, 65, 67) umfassen, die sich diametral gegenüberliegend an einer Schaftinnenfläche durch den Schaft (3, 47, 137) erstrecken, wobei der Schaft (3, 47, 137) in einem krümmbaren Schaftabschnitt (9, 49) eine Mehrzahl von Schlitzen (33, 55, 64) mit axialen Abständen zueinander aufweist, wobei sich die Schlitze (33, 55, 64) in Umfangsrichtung nur über einen Teil des Schaftumfangs erstrecken, und wobei die Schlitze (33, 55, 64) axial zueinander derart angeordnet sind, dass sie abwechselnd auf einer ersten lateralen Seite des Schafts (3, 47, 137) und einer diametral der ersten gegenüberliegenden zweiten lateralen Seite des Schafts (3, 47, 137) liegen, wobei der krümmbare Schaftabschnitt (9, 49) mindestens zwei Unterabschnitte (Y, Z) aufweist, wobei die Abstände zwischen den Schlitzen (33, 55, 64) in einem ersten Unterabschnitt (Y) der mindestens zwei Unterabschnitte (Y, Z) kleiner ist als in einem zweiten Unterabschnitt (Z) der mindestens zwei Unterabschnitte (Y, Z), wobei der erste Unterabschnitt (Y) distal vom zweiten Unterabschnitt (Z) angeordnet ist, **dadurch gekennzeichnet dass** die Schlitze (33, 55, 64) keilförmig ausgestaltet sind, sodass die lichte Weite der Schlitze (33, 55, 64) mittig, wo die Zugdrähte (57, 59, 65, 67) verlaufen, am größten ist und sich zu den Enden hin in Umlaufrichtung verjüngt, wobei die Enden der Schlitze (33, 55, 64) Rundausnehmungen aufweisen, wobei die lichte Weite der Schlitze (33, 55, 64) im ersten Unterabschnitt (Y) größer ist als im zweiten Unterabschnitt (Z).

2. Endoskopisches Instrument (1,134) nach Anspruch 1, wobei im Schaft (3, 47, 137) ein die mindestens zwei durch den Schaft (3, 47, 137) verlaufenden Leitungen (11, 13, 15, 19, 57, 59, 65, 67, 123, 181) jeweils einzeln unmittelbar umgebender Fluidkanal (7, 53) ausgebildet ist.

3. Endoskopisches Instrument (1,134) nach Anspruch 2, wobei die Schlitze (33, 55, 64) einerseits als eine distale Fluidkanalöffnung für den Fluidkanal (7, 53) und andererseits zur lokalen Erhöhung der Flexibilität des Schafts (3, 47, 137) zum Abwinkeln eines distalen Schaftendes (17, 51, 139) dienen.

4. Endoskopisches Instrument (1,134) nach Anspruch 2 oder3, wobei der Fluidkanal (7, 53) als Zu- und/oder Abführkanal dient.

5. Endoskopisches Instrument (1,134) nach einem der Ansprüche 2 bis 4, wobei der Fluidkanal (7, 53) eine distale Fluidkanalöffnung (33, 55, 64, 140, 203) und eine proximale Fluidkanalöffnung (45, 73, 142) aufweist, wobei die proximale Fluidkanalöffnung (45, 73, 142) lateral am Schaft (3, 47, 137) angeordnet und mit Fluiddruck oder Fluidunterdruck beaufschlagbar ist.

6. Endoskopisches Instrument (1,134) nach einem der vorhergehenden Ansprüche, wobei das Instrument (1,134) ein Dichtmittel (89) aufweist, das ein proximales Ende des Fluidkanals (7, 53) bildet und Durchführungen (93, 97, 99, 101, 103) für die Leitungen (11, 13, 15, 19, 57, 59, 65, 67, 123, 181) aufweist.

7. Endoskopisches Instrument (1,134) nach einem der vorhergehenden Ansprüche, wobei das Instrument (1,134) eine Handhabungseinrichtung (5, 135) aufweist, wobei die Handhabungseinrichtung (5, 135) mit einem proximalen Ende (4, 138) des Schafts (3, 47, 137) fest verbunden oder lösbar verbindbar ist.

8. Endoskopisches Instrument (1,134) nach einem der vorhergehenden Ansprüche, wobei der Schaft (3, 47, 137) zumindest in dem krümmbaren Schaftabschnitt (9, 49) flexibel oder semi-flexibel ist.

9. Endoskopisches Instrument (1, 134) nach einem der vorhergehenden Ansprüche, wobei das Instrument (1,134) oder zumindest der Schaft (3, 47, 137) als Verbrauchsartikel zum Entsorgen nach einmaliger Verwendung ausgestaltet ist.

10. Endoskopisches Instrument (1,134) nach einem der vorhergehenden Ansprüche, wobei die Querschnittsfläche des Fluidkanals (7, 53) der Querschnittsfläche eines vom Schaft (3, 47, 137) gebildeten Schaftinnenraums abzüglich der Summe der Querschnittsflächen aller durch den Schaftinnenraum verlaufenden Leitungen (11, 13, 15, 19, 57, 59, 65, 67, 123, 181) entspricht.

11. Endoskopisches Instrument (1,134) nach einem der vorhergehenden Ansprüche, wobei das Instrument (1,134) mindestens einen durch den Schaft (3, 47, 137) verlaufenden und vom Fluidkanal (7, 53) unmittelbar umgebenen Arbeitskanal (15, 19, 131) aufweist.

12. Endoskopisches Instrument (1,134) nach Anspruch 11, wobei der Arbeitskanal (15, 19, 131) zur Durchführung eines Laserlichtleiters (123), eines Fangkorbes (191) oder einer Dormia-Schlinge dient.

13. Endoskopisches Instrument (1,134) nach Anspruch 11 oder 12, wobei der Arbeitskanal (15, 19, 131) als Zu- und/oder Abführkanal (19) mit gegenüber dem Fluidkanal (7, 53) gegenläufiger Strömungsrichtung dient.

14. Endoskopisches Instrument (1,134) nach einem der Ansprüche 11 bis 13, wobei der Arbeitskanal (15, 19, 131) axial durch ein abgedichtetes proximales Ende des Fluidkanals (7, 53) verläuft.

15. Endoskopisches Instrument (1,134) nach einem der Ansprüche 11 bis 14, wobei der Arbeitskanal (15, 19, 131) eine distale Arbeitskanalöffnung (18, 52, 140, 211) und eine proximale Arbeitskanalöffnung (39, 144, 153) aufweist.

## Claims

1. Endoscopic instrument (1, 134) for introduction into a body of a patient, wherein the instrument (1, 134) comprises a tubular shaft (3, 47, 137) and at least two electrical, mechanical and/or optical lines (11, 13, 15, 19, 57, 59, 65, 67, 123, 181) extending through the shaft (3, 47, 137), wherein the lines (11, 13, 15, 19, 57, 59, 65, 67, 123, 181) comprise at least two tension wires (57, 59, 65, 67) which extend in a diametrically opposed manner though the shaft (3, 47, 137) on an inner surface of the shaft,
wherein the shaft (3, 47, 137) in a bendable shaft section (9, 49) has a plurality of slits (33, 55, 64) at an axial distance with regard to each other, wherein in the circumferential direction, the slits (33, 55, 64) only extend over a part of the shaft circumference and wherein the slits (33, 55, 64) are axially arranged in relation to each other so that alternately they lie on a first lateral side of the shaft (3, 47, 137) and on a second lateral side of the shaft (3, 47, 137) which is diametrically opposite the first lateral side, wherein the bendable shaft section (9, 49) has at least two sub-sections (Y, Z), wherein the distances between the slits (33, 55, 64) in a first sub-section (Y) of the at least two sub-sections (Y, Z) is smaller than in a second sub-section (Z) of the at least two sub-sections (Y, Z), wherein the first sub-section (Y) is arranged distally of the second sub-section (Z),
**characterised in that** that slits (33, 55, 64) are wedge-shaped so that the clear width of the slits (33, 55, 64) centrally, where the tension wires (57, 59, 65, 67) run is largest and narrow towards the ends in the circumferential direction, wherein the ends of the slits (33, 55, 64) have round recesses, wherein the clear width of the slits (33, 55, 64) in the first sub-section (Y) is greater than in the second sub-section (Z).

2. Endoscopic instrument (1, 134) according to claim 1, wherein in the shaft (3, 47, 137) there is a fluid channel (7, 53) that directly surrounds each one of the at least two lines (11, 13, 15, 19, 57, 59, 65, 67, 123, 181) that extend through the shaft (3, 47, 137).

3. Endoscopic instrument (1, 134) according to claim 2, wherein the slits (33, 55, 64) on the one hand serve as a distal fluid channel opening for the fluid channel (7, 53) and on the other hand to locally increase the flexibility of the shaft (3, 47, 137) for angling a distal end of the shaft (17, 51, 139).

4. Endoscopic instrument (1, 134) according to claim 2 or 3, wherein the fluid channel (7, 53) serves as a supply and/or discharge channel.

5. Endoscopic instrument (1, 134) according to any one of claims 2 to 4, wherein the fluid channel (7, 53) has a distal fluid channel opening (33, 55, 64, 140, 203) and a proximal fluid channel opening (45, 73, 142), wherein the proximal fluid channel opening (45, 73, 142) is arranged laterally on the shaft (3, 47, 137) and fluid pressure or fluid under-pressure can act on it.

6. Endoscopic instrument (1, 134) according to any one of the preceding claims, wherein the instrument (1, 134) comprises a sealing means (89) which forms a proximal end of the fluid channel (7, 53) and has ducts (93, 97, 99, 101, 103) for the lines (11, 13, 15, 19, 57, 59, 65, 67, 123, 181).

7. Endoscopic instrument (1, 134) according to any one of the preceding claims, wherein the instrument (1, 134) has a manipulation device (5, 135), wherein the manipulation device (5, 135) is firmly connected to or can be detachably connected to a proximal end (4, 138) of the shaft (3, 47, 137).

8. Endoscopic instrument (1, 134) according to any one of the preceding claims wherein the shaft (3, 47, 137), at least in the bendable shaft section (9, 49), is flexible or semi-flexible.

9. Endoscopic instrument (1, 134) according to any one of the preceding claims, wherein the instrument (1, 134) or at least the shaft (3, 47, 137) is designed as a disposable article to be discarded after a single use.

10. Endoscopic instrument (1, 134) according to any one of the preceding claims, wherein the cross-sectional area of the fluid channel (7, 53) corresponds to the cross-sectional area of a shaft interior space formed by the shaft (3, 47, 137) minus the sum of the cross-sectional areas of all the lines (11, 13, 15, 19, 57, 59, 65, 67, 123, 181) extending through the interior space of the shaft.

11. Endoscopic instrument (1, 134) according to any one of the preceding claims, wherein the instrument (1, 134) has an operating channel (15, 19, 131) that extends through the shaft (3, 47, 137) and is directly surrounded by the fluid channel (7, 53).

12. Endoscopic instrument (1, 134) according to claim 11, wherein the operating channel (15, 19, 131) is used for passing through a laser light guide (123), a collection basket (191) or a Dormia's loop.

13. Endoscopic instrument (1, 134) according to claim 11 or 12, wherein the operating channel (15, 19, 131) serves as a supply and/or discharge channel (19) with a flow direction counter to that of the fluid channel (7, 53).

14. Endoscopic instrument (1, 134) according to any one of claims 11 to 13, wherein the operating channel (15 ,19, 131) extends axially through a sealed off proximal end of the fluid channel (7, 53).

15. Endoscopic instrument (1, 134) according to any one of claims 11 to 14, wherein the operating channel (15, 19, 131) comprises a distal operating channel opening (18, 52, 140, 211) and a proximal operating channel opening (39, 144, 153).

## Revendications

1. Instrument endoscopique (1, 134) destiné à être introduit dans un corps d'un patient, dans lequel l'instrument (1, 134) présente un arbre tubulaire (3, 47, 137) et au moins deux lignes électriques, mécaniques et/ou optiques (11, 13, 15, 19, 57, 59, 65, 67, 123, 181) passant à travers l'arbre (3, 47, 137), dans lequel les lignes (11, 13, 15, 19, 57, 59, 65, 67, 123, 181) comprennent au moins deux câbles de traction (57, 59, 65, 67) qui s'étendent de manière diamétralement opposée sur une face intérieure d'arbre à travers l'arbre (3, 47, 137),
dans lequel l'arbre (3, 47, 137) présente dans un tronçon d'arbre pouvant être fléchi (9, 49), une pluralité de fentes (33, 55, 64) avec des écarts axiaux entre elles, dans lequel les fentes (33, 55, 64) s'étendent uniquement sur une partie du périmètre de l'arbre dans le sens périphérique, et dans lequel les fentes (33, 55, 64) sont ainsi disposées axialement entre elles qu'elles reposent en alternance sur un premier côté latéral de l'arbre (3, 47, 137) et un deuxième côté latéral de l'arbre (3, 47, 137) diamétralement opposé au premier, dans lequel le tronçon d'arbre pouvant être fléchi (9, 49) présente au moins deux sous-tronçons (Y, Z), dans lequel les écarts entre les fentes (33, 55, 64) dans un premier sous-tronçon (Y) des au moins deux sous-tronçons (Y, Z) sont plus petits que dans un deuxième sous-tronçon (Z) des au moins deux sous-tronçons (Y, Z), dans lequel le premier sous-tronçon (Y) est disposé de manière distale au deuxième sous-tronçon (Z),
**caractérisé en ce que**
les fentes (33, 55, 64) sont cunéiformes de façon à ce que le diamètre intérieur des fentes (33, 55, 64) est le plus gros au centre, là où les câbles de traction (57, 59, 65, 67) passent et se réduit dans le sens périphérique en direction des extrémités, dans lequel les extrémités des fentes (33, 55, 64) présentent des évidements ronds, dans lequel le diamètre intérieur des fentes (33, 55, 64) dans le premier sous-tronçon (Y) est plus grand que dans le deuxième sous-tronçon (Z).

2. Instrument endoscopique (1, 134) selon la revendication 1, dans lequel un canal fluidique (7, 53) entourant respectivement directement et individuellement les au moins deux lignes (11, 13, 15, 19, 57, 59, 65, 67, 123, 181) passant à travers l'arbre (3, 47, 137), est formé dans l'arbre (3, 47, 137).

3. Instrument endoscopique (1, 134) selon la revendication 2, dans lequel les fentes (33, 55, 64) servent d'une part en tant qu'une ouverture distale de canal fluidique pour le canal fluidique (7, 53) et d'autre part pour augmenter localement la flexibilité de l'arbre (3, 47, 137) pour couder une extrémité d'arbre distale (17, 51, 139).

4. Instrument endoscopique (1, 134) selon la revendication 2 ou 3, dans lequel le canal fluidique (7, 53) sert de canal d'alimentation et/ou d'évacuation.

5. Instrument endoscopique (1, 134) selon l'une des revendications 2 à 4, dans lequel le canal fluidique (7, 53) présente une ouverture de canal fluidique distale (33, 55, 64, 140, 203) et une ouverture de canal fluidique proximale (45, 73, 142), dans lequel l'ouverture de canal fluidique proximale (45, 73, 142) est disposée latéralement sur l'arbre (3, 47, 137) et peut être alimentée en pression fluidique ou sous-pression fluidique.

6. Instrument endoscopique (1, 134) selon l'une des revendications précédentes, dans lequel l'instrument (1, 134) présente un moyen d'étanchéité (89) qui forme une extrémité proximale du canal fluidique (7, 53) et des passages (93, 97, 99, 101, 103) pour les lignes (11, 13, 15, 19, 57, 59, 65, 67, 123, 181).

7. Instrument endoscopique (1, 134) selon l'une des revendications précédentes, dans lequel l'instrument (1, 134) présente un dispositif de manipulation (5, 135), dans lequel le dispositif de manipulation (5, 135) est relié fixement ou peut être relié de manière séparable à une extrémité proximale (4, 138) de l'arbre (3, 47, 137).

8. Instrument endoscopique (1, 134) selon l'une des revendications précédentes, dans lequel l'arbre (3, 47, 137) est au moins flexible ou semi-flexible au moins dans le tronçon d'arbre pouvant être fléchi (9, 49).

9. Instrument endoscopique (1, 134) selon l'une des revendications précédentes, dans lequel l'instrument (1, 134) ou au moins l'arbre (3, 47, 137) est conçu en tant qu'article consommable destiné à être mis au rebut après usage unique.

10. Instrument endoscopique (1, 134) selon l'une des revendications précédentes, dans lequel la surface transversale du canal fluidique (7, 53) correspond à la surface transversale d'un espace intérieur d'arbre formé par l'arbre (3, 47, 137) moins la somme des surfaces transversales de toutes les lignes (11, 13, 15, 19, 57, 59, 65, 67, 123, 181) passant à travers l'espace intérieur d'arbre.

11. Instrument endoscopique (1, 134) selon l'une des revendications précédentes, dans lequel l'instrument (1, 134) présente au moins un canal de travail (15, 19, 31) passant à travers l'arbre (3, 47, 137) et entouré directement par le canal fluidique (7, 53).

12. Instrument endoscopique (1, 134) selon la revendication 11, dans lequel le canal de travail (15, 19, 31) sert à faire passer un guide de lumière laser (123), un piège (191) ou une sonde de Dormia.

13. Instrument endoscopique (1, 134) selon la revendication 11 ou 12, dans lequel le canal de travail (15, 19, 31) sert en tant que canal d'alimentation et/ou d'évacuation (19) avec un sens d'écoulement contraire par rapport au canal fluidique (7, 53).

14. Instrument endoscopique (1, 134) selon l'une des revendications 11 à 13, dans lequel le canal de travail (15, 19, 31) passe axialement à travers une extrémité proximale étanchéifiée du canal fluidique (7, 53).

15. Instrument endoscopique (1, 134) selon l'une des revendications 11 à 14, dans lequel le canal de travail (15, 19, 31) présente une ouverture de canal de travail distale (18, 52, 140, 211) et une ouverture de canal de travail proximale (39, 144, 153).
